# EUROPEAN PATENT SPECIFICATION

(11) **EP 2 081 536 B1**
(45) Date of publication and mention of the grant of the patent: **24.07.2013**
(21) Application number: 07849155.2
(22) Date of filing: 15.11.2007
(51) Int. Cl.: A61F 13/56

(54) **DISPOSABLE ABSORBENT ARTICLE HAVING A WRAP AND TUCK CONFIGURATION**
SAUGFÄHIGER EINWEGARTIKEL MIT WICKEL- UND STECK-KONFIGURATION
ARTICLE ABSORBANT JETABLE DU TYPE ENVELOPPANT, À RABAT

(30) Priority: 15.11.2006 US 599852; 11.09.2007 US 900311
(43) Date of publication of application: 29.07.2009
(73) Proprietor: THE PROCTER & GAMBLE COMPANY, Cincinnati, OH 45202 (US)
(72) Inventor: ROE, Donald Carroll, West Chester, Ohio 45069 (US); BERGMAN, Carl Louis, Loveland, Ohio 45140 (US); DREIER, Kimberly Ann, Cincinnati, Ohio 45251 (US); FISHER, Constance Lee, Cincinnati, Ohio 45246 (US); FEIST, Barry Robert, Madeira, Ohio 45243 (US); KLINE, Mark James, Okeana, OH 45053 (US)
(74) Representative: Heide, Ute
(86) International application number: PCT/IB2007/054663
(87) International publication number: WO 2008/059461

(56) References cited:
- GB-A- 2 263 224
- US-A- 3 089 494
- US-A- 4 995 873

## Description

### FIELD OF THE INVENTION

The present disclosure relates to disposable absorbent articles, and more particularly, to disposable absorbent articles having wrap and tuck folding configurations for application of the articles to wearers that mimic swaddling characteristics.

### BACKGROUND OF THE INVENTION

Disposable absorbent articles having various different basic designs are known in the art. Infants and other incontinent individuals may wear absorbent articles such as diapers and incontinent briefs to receive and contain discharged urine and other body exudates. Such absorbent articles function both to contain the discharged materials and to isolate bodily exudates from the body of the wearer and from the wearer's garments and bed clothing.

Some disposable absorbent articles are provided in a form that requires assembly of the article relative to the body of the wearer during the application process. Such disposable diapers may be configured to appeal to mothers and babies in various stages of development. One such stage, referred to herein as the "bonding stage," contemplates the relationship between mothers and newborn babies. Some mothers of newborn infants have a desire to wrap their newborn in blankets and/or other fabrics to help provide a feeling of comfort and security. The traditional means of wrapping a child is called swaddling. The overlapping and wrapping of blankets (e.g. swaddling) comforts the baby and provides a close interaction with the baby for the mother. As such, "blanket-like" product forms may be aspirational and intuitive for mothers with babies in this stage. Some diapers may be configured to mimic as much of this swaddling characteristic as possible, such as the absorbent garment disclosed in U.S. Pat. No. 4,995,873, issued to Knight.

Some absorbent articles, such as the absorbent garment of Knight, configured to mimic swaddling characteristics lack various features, such as stretchable components (e.g. ears, waist, chassis), that would otherwise enhance the fit, comfort, and basic diaper function of waste containment. These missing features are important to enable conformation to a child's body and would otherwise allow the absorbent article to easily adapt to a child's shape and provide comforting pressure to a child. Further, some such absorbent articles may have pre-formed shapes that are relatively complex to manufacture and that require relatively more material to construct, resulting in higher manufacturing costs.

### SUMMARY OF THE INVENTION

Aspects of the present disclosure involve a disposable absorbent article configured to be placed on the body of a wearer to mimic swaddling characteristics. The disposable absorbent article may include a chassis having a tuck flap and two ears or side panels. The two ears extend in opposing lateral directions from a first waist region, and the tuck flap extends in a longitudinal direction from a second waist region. Other chassis embodiments may include two additional ears extending in opposing lateral directions from the second waist region. The chassis is adapted to fit a wearer by wrapping the two ears around a wearer's waist, passing the second waist region between a wearer's legs, and folding and/or tucking the tuck flap over the ears. The ears may be adapted to connect with each other and/or another portion of the chassis, such as the tuck flap and/or the second waist region to hold the diaper in position on the wearer's body. In addition, embodiments of the chassis may be constructed with a pre-formed tuck flap that may also be stretchable. Some chassis embodiments may be constructed from materials that allow a user to manually form a tuck flap by longitudinally stretching a portion of the second waist region of the chassis.

In one form, a disposable absorbent article adapted to be worn about a lower torso region of a wearer includes: a chassis having an outer surface and an inner surface and including a first waist region, a second waist region, and a crotch region disposed intermediate the first waist region and the second waist region, the chassis further including a first ear, a second ear, and a tuck flap, wherein the first and second ears extend in opposing lateral directions from the first waist region and the tuck flap extends in a longitudinal direction from the second waist region; at least one fastener element disposed on a first area of the chassis and adapted to connect with a second area of the chassis; and wherein at least one portion of the tuck flap is stretchable.

In another form, a disposable absorbent article adapted to be worn about a lower torso region of a wearer includes: a chassis having an outer surface and an inner surface and including a first waist region, a second waist region, and a crotch region disposed intermediate the first waist region and the second waist region, the chassis further including a first ear, a second ear, and a tuck flap, wherein the first and second ears extend in opposing lateral directions from the first waist region and the tuck flap extends in a longitudinal direction from the second waist region; at least one fastener element disposed on a first area of the chassis and adapted to connect with a second area of the chassis; and wherein the first and second ears are stretchable.

In yet another aspect, a method for placing a disposable absorbent article on a lower torso region of a wearer includes the steps of: positioning a first waist region of a chassis adjacent a wearer's rear waist area; wrapping the first and second ears extending from the first waist region around a wearer's waist; passing a second waist region and a crotch region of the chassis between a wearer's legs; engaging the first and second ears with the second waist region of the chassis adjacent a wearer's front waist area; stretching a tuck flap extending from the second waist region of the chassis; and folding the tuck flap over the first and second ears.

In yet another form, a disposable absorbent article adapted to be worn about a lower torso region of a wearer includes: a chassis having an outer surface and an inner surface and including a first waist region, a second waist region, and a crotch region disposed intermediate the first waist region and the second waist region, the chassis further including a first ear, a second ear, and a tuck flap, wherein the first and second ears extend in opposing lateral directions from the first waist region and the tuck flap extends in a longitudinal direction from the second waist region, and wherein the first ear defines a first ear area on the inner surface and a first ear area on the outer surface; fastening elements disposed on the first ear and adapted to connect with a second area of the chassis; and wherein at least about 25% of either the first ear area on the inner surface or the first ear area on the outer surface is covered with the fastening elements.

In still another form, a disposable absorbent article adapted to be worn about a lower torso region of a wearer includes: a chassis having an outer surface and an inner surface and including a first waist region, a second waist region, and a crotch region disposed intermediate the first waist region and the second waist region, the chassis further including a first ear, a second ear, and a tuck flap, wherein the first and second ears extend in opposing lateral directions from the first waist region and the tuck flap extends in a longitudinal direction from the second waist region, wherein the tuck flap defines a tuck flap area on the inner surface and a tuck flap area on the outer surface; fastening elements disposed on the tuck flap and adapted to connect with a second area of the chassis; and wherein at least 25% either the tuck flap area on the inner surface or the tuck flap area on the outer surface is covered by the fastening elements.

In still another form, a disposable absorbent article adapted to be worn about a lower torso region of a wearer includes: a chassis having an outer surface and an inner surface and including a first waist region, a second waist region, and a crotch region disposed intermediate the first waist region and the second waist region, the chassis further including a first ear, a second ear, and a tuck flap, wherein the first and second ears extend in opposing lateral directions from the first waist region and the tuck flap extends in a longitudinal direction from the second waist region, wherein the chassis defines a first chassis area on the inner surface and a second chassis area on the outer surface; fastening elements disposed on the chassis and adapted to connect with either the tuck flap, the first ear, or the second ear; and wherein at least 25% either the first chassis area or the second chassis area is covered by the fastening elements.

### BRIEF DESCRIPTION OF THE DRAWINGS

Fig. 1 is a top plan view of a disposable absorbent article in accordance with the present disclosure.
Fig. 1A is a view of the disposable article of Fig. 1 showing additional details of the ears and tuck flap.
Fig. 2 is an isometric view of the disposable absorbent article placed on a wearer's body with a tuck flap folded over first and second ears.
Fig. 3A is a top plan view of a disposable absorbent article having a stretchable second waist region.
Fig. 3B is a top plan view of the disposable absorbent article of Fig. 3A with a tuck flap manually formed by stretching the second waist region.
Fig. 4A is a top plan view of a disposable absorbent article having two pairs of ears and a stretchable second waist region.
Fig. 4B is a top plan view of the disposable absorbent article of Fig. 4A with a tuck flap manually formed by stretching the second waist region.
Fig. 5A is an isometric view of a disposable absorbent article in the process of being folded as when being placed on a wearer.
Fig. 5B is an isometric view of a disposable absorbent article in the process of being folded as when being placed on a wearer.
Fig. 5C is an isometric view of a disposable absorbent article in the process of being folded with first and second ears overlapped across the second waist region of the chassis.
Fig. 5D is an isometric view of a disposable absorbent article in the process of being folded with first and second ears in extending across the second waist region.
Fig. 6A is a top plan view of a disposable absorbent article showing various fastener element locations on an inner, body-facing surface of the chassis.
Fig. 6B is a bottom plan view of a disposable absorbent article showing various fastener element locations on an outer, garment-facing surface of the chassis.
Fig. 7A is a top plan view of a disposable absorbent article having two pairs of ears and showing various fastener element locations on an inner, body-facing surface of the chassis.
Fig. 7B is a bottom view of a disposable absorbent article showing various fastener element locations on an outer, garment-facing surface of the chassis.
Fig. 8 is an isometric view of the disposable absorbent article placed on a wearer's body with first and second ears extending across a portion of the second waist region of the chassis.
Fig. 9 is an isometric view of the disposable absorbent article having third and fourth ears and placed on a wearer's body.
Fig. 10 is an isometric view of the disposable absorbent article placed on a wearer's body with the tuck flap extending between the wearer's body and the first and second ears.

### DETAILED DESCRIPTION OF THE INVENTION

Aspects of the present disclosure involve a disposable absorbent article in the form of a diaper configured to be placed on the body of wearer in a "wrap and tuck" configuration to mimic swaddling characteristics. Embodiments of the disposable absorbent article include a chassis having a tuck flap and two ears or side panels. The chassis also includes a first waist region, a second waist region, and a crotch region longitudinally disposed intermediate the first and second waist regions. The two ears extend in opposing lateral directions from the first waist region, and the tuck flap extends in a longitudinal direction from the second waist region. As such, some embodiments of the chassis define a T-shaped form when the chassis is placed in a flat, unfolded configuration. Other chassis embodiments may include two additional ears extending in opposing lateral directions from the second waist region to define a more conventional form when placed in a flat, unfolded configuration. The chassis is adapted to fit a wearer by wrapping the two ears around a wearer's waist region, passing the second waist region through a wearer's crotch region, and folding the tuck flap over the ears and/or tucking the tuck flap between the chassis and the wearer's body. The ears may be adapted to connect with each other and/or another portion of the chassis, such as the tuck flap and/or the second waist or crotch region to hold the diaper in position on the wearer's body.

As discussed in more detail below, embodiments of the chassis may be constructed with a pre-formed tuck flap longitudinally extending from the second waist region. The pre-formed tuck flap may also be stretchable. Other chassis embodiments may not include a pre-formed tuck flap and instead may be constructed from materials that allow a user to manually form a tuck flap by stretching (elastically or by permanently deforming) a portion of the second waist region of the chassis. As such, the tuck flap may be stretchable, extensible, elastic. A chassis configured with a tuck flap may provide various benefits. For example, a user can adjust a chassis to fit a wide range of wearer sizes by adjusting how the tuck flap is connected with other portions of the chassis. As such, a chassis configured with a tuck flap may adjusted to accommodate a baby's rapid growth. In addition, the tuck flap can be easily positioned to avoid a baby's umbilical stump, which helps prevent irritation to the baby and provide additional comfort thereto. Embodiments of the disposable absorbent article may also be configured with various features that may help provide improved comfort, fit, and/or waste containment as well as reduced manufacturing complexity. For example, some embodiments may include stretchable ears, an apertured topsheet, leg cuffs, and/or various absorbent core configurations. In addition, embodiments may include a waste management element including acceptance and storage elements or an elasticized topsheet having an opening adapted to receive feces, as referenced below.

As used herein, the term "diaper" refers to an absorbent article generally worn by infants and incontinent persons about the lower torso. The term "absorbent article" refers to devices which absorb and contain body exudates, and more specifically, refers to devices which are placed against or in proximity to the body of the wearer to absorb and contain the various exudates discharged from the body. The term "disposable" is used herein to describe absorbent articles which generally are not intended to be laundered or otherwise restored or reused as an absorbent article (e.g., they are intended to be discarded after a single use and may also be configured to be recycled, composted or otherwise disposed of in an environmentally compatible manner). As used herein, the term "disposed" is used to mean that an element(s) of the diaper is formed (joined and positioned) in a particular place or position as a unitary structure with other elements of the diaper or as a separate element joined to another element of the diaper. As used herein, the term "joined" encompasses configurations whereby an element is directly secured to another element by affixing the element directly to the other element, and configurations whereby an element is indirectly secured to another element by affixing the element to intermediate member(s) which in turn are affixed to the other element.

As used herein the term "stretchable" refers to materials which can stretch to at least an elongated length of 105% on the upcurve of the hysteresis test at a load of about 400 gm/cm. The term "non-stretchable" refers to materials which cannot stretch to at least 5% on the upcurve of the hysteresis test at a load of about 400 gm/cm.

The terms "elastic" and "elastomeric" as used herein refer to any material that upon application of a biasing force, can stretch to an elongated length of at least about 110%, preferably to 125% of its relaxed, original length (i.e. can stretch to 10 percent, preferably 25% more than its original length), without rupture or breakage, and upon release of the applied force, recovers at least about 40% of its elongation, preferably recovers at least 60% of its elongation, most preferably recovers at least about 80% of its elongation. For example, a material that has an initial length of 100 mm can extend at least to 110 mm, and upon removal of the force would retract to a length of 106 mm (40% recovery). The term "inelastic" refers herein to any material that does not fall within the definition of "elastic" above.

The term "extensible" as used herein refers to any material that upon application of a biasing force, can stretch to an elongated length of at least about 110%, preferably 125% of its relaxed, original length (i.e. can stretch to 10 percent, preferably 25% more than its original length), without rupture or breakage, and upon release of the applied force, shows little recovery, less than about 40% preferably less than about 20% and more preferably less than about 10% of its elongation.

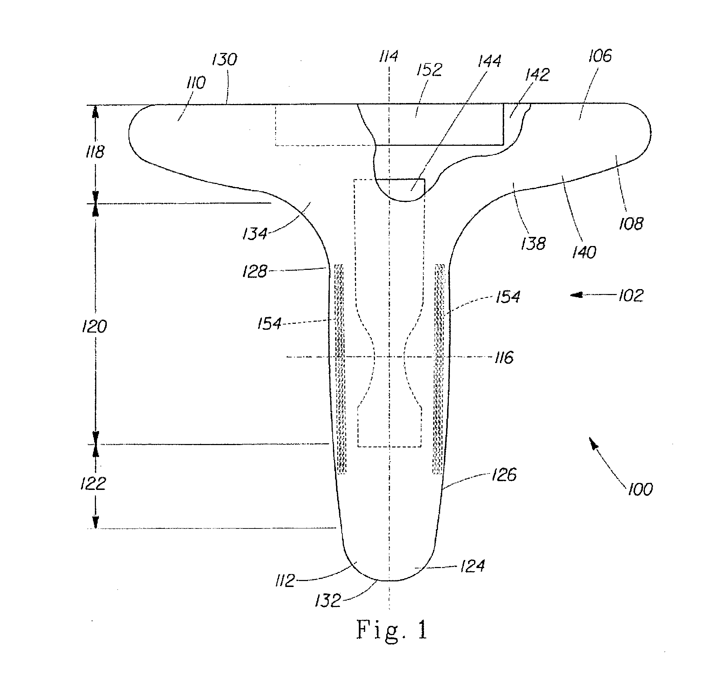

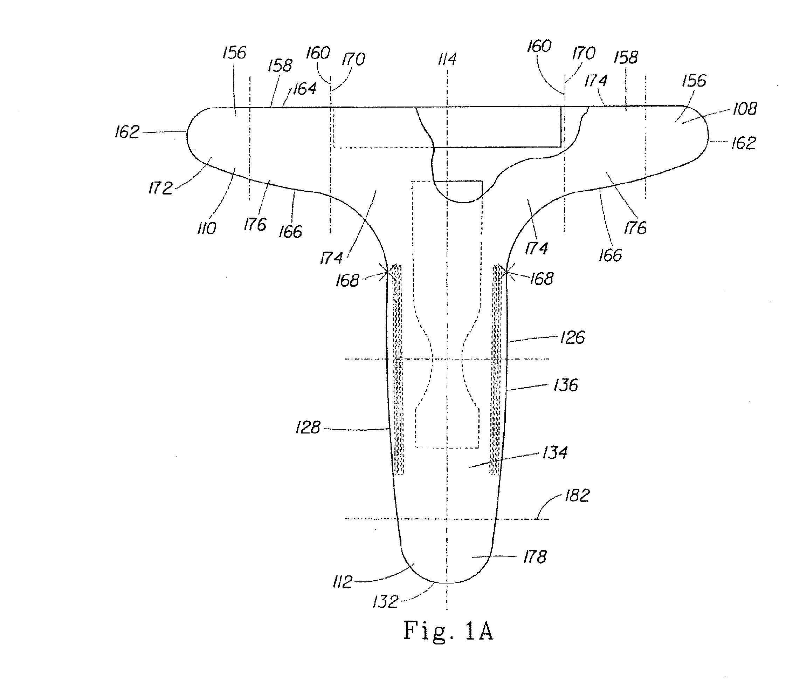

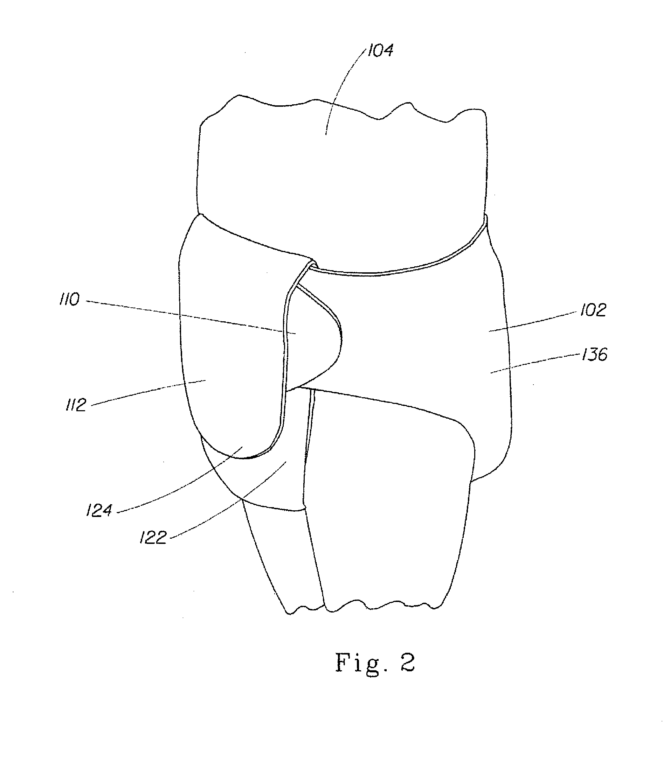

Figs. 1 and 2 show an embodiment of a disposable absorbent article 100 in the form of a diaper 102. In particular, Fig. 1 is a plan view of the diaper 102 in a flat, unfolded condition, with the portion of the diaper that faces a wearer oriented towards the viewer, and Fig. 2 shows the diaper 102 of Fig. 1 in a folded state and placed on a wearer's body 104. As discussed in more detail below, it is possible to wrap and fold the diaper 102 on a wearer's body in other ways than as shown in Fig. 2. As shown in Fig. 1, the diaper includes a chassis 106 having a first ear 108, a second ear 110, and a tuck flap 112. To provide a frame of reference for the present discussion, the chassis is shown with a longitudinal axis 114 and a lateral axis 116. The chassis 106 is shown as having a first waist region 118, a second waist region 122, and a crotch region 120 disposed intermediate the first and second waist regions. The tuck flap 112 extends longitudinally from the second waist region 122. As such, the chassis 106 shown in Fig. 1 has a general T-shaped form defined by the laterally extending first ear 108 and second ear 110 and the longitudinally extending tuck flap 112, second waist region 122, and crotch region 120. As shown in Fig. 1, the tuck flap 112 extends from the second waist region 122 to a distal end portion 124. The periphery of the diaper is defined by a pair of longitudinally extending side edges 126, 128; a first outer edge 130 extending laterally adjacent the first waist region 118; and a second outer edge 132 extending laterally adjacent the distal end portion 124 of the tuck flap 112.

As shown in Figs. 1 and 2, the chassis 106 includes an inner, wearer or body-facing surface 134, and an outer, garment-facing surface 136. A portion of the chassis structure is cut-away in Fig. 1 to more clearly show the construction of and various features that may be included in embodiments of the diaper. As shown in Fig. 1, the chassis 106 of the diaper 102 may include an outer covering layer 138 including a topsheet 140 and a backsheet 142. An absorbent core 144 may be disposed between a portion of the topsheet 140 and the backsheet 142. As discussed in more detail below, any one or more of the regions may be stretchable and may include an elastomeric material or laminate as described herein. As such, the diaper 102 may be configured to adapt to a specific wearer's anatomy upon application and to maintain coordination with the wearer's anatomy during wear.

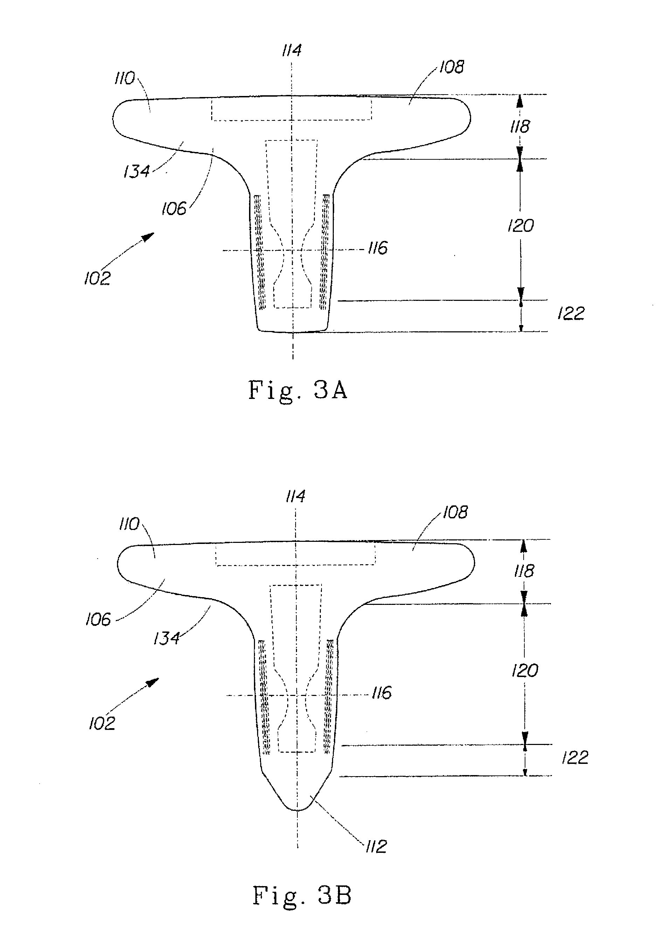

As previously mentioned, the tuck flap 112 of the diaper 102 may be pre-formed and may also be constructed of materials that allow the tuck flap 112 to be stretched. In other embodiments, the tuck flap 112 may be manually formed by stretching portions of the chassis 106 in longitudinal and/or lateral directions, such as from the second waist region 122. For example, Figs. 3A and 3B illustrate an embodiment of the chassis 106 wherein the tuck flap 112 is manually formed by stretching a portion of the chassis 106. In particular, the chassis is shown in Fig. 3A with the first waist region 118, crotch region 120, and second waist region 122, similarly shown in Fig. 1. However, unlike Fig. 1, the chassis 106 in Fig. 3A is shown without the tuck flap 112. One or more portions of the chassis 106, such as the second waist region 122, in Figs. 3A and 3B may constructed from materials that allow the tuck flap 112 to be manually formed by exerting forces on the second waist region 122 in longitudinal and/or lateral directions. One example of the manually formed tuck flap 112 is shown in Fig. 3B.

It is to be appreciated that the manually formed tuck flap may have shapes and sizes other than those illustrated in the figures. For example, in some embodiments, it may be preferable to have a tuck flap that, when folded over the ears, defines a length from the fold to a distal end of the tuck flap (i.e. the folded length) in the range of 1 cm to 15 cm; while in other embodiments it may be preferable to have a tuck flap defining a folded length in the range of 2 cm to 10 cm; and yet in other embodiments it may be preferable to have a tuck flap defining a folded length in the range of 2.5 cm to 5 cm. It is also to be appreciated that the manually formed tuck flaps may be formed before, during, and/or after placing the diaper on the wearer. In addition, diapers according to the present disclosure may also include a combination of pre-formed and manually formed tuck flaps, wherein a pre-formed tuck flap can be manually by the user stretched to a desired shape and/or length. A discussion of various types of stretchable or elastic materials that may used to construct the chassis to provide for the manually formable tuck flap is provided below. Although the tuck flap 112 is illustrated as being integrally formed with the chassis 106, it is to be appreciated that other embodiments may include a tuck flap that is a discrete element connected with the chassis.

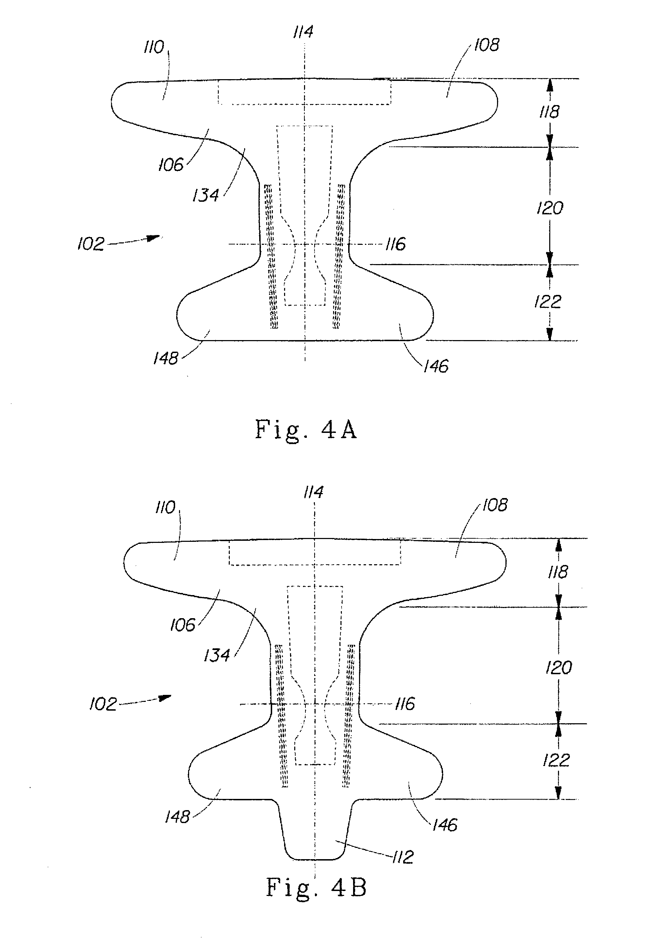

It is to be appreciated that the diaper 102 may have various features that define chassis shapes other than the general T-shaped form shown in Fig. 1. For example, Figs. 4A and 4B show the chassis 106 with a third ear 146 and a fourth ear 148 extending in opposing lateral directions from the second waist region 122. As such, the chassis 106 shown in Fig. 4A defines a more conventional form when placed in a flat, unfolded configuration. The second waist region 122 of the chassis 106 shown in Fig. 4A may also be constructed from materials that allow the tuck flap 112 to be manually formed by exerting forces on portions of the chassis 106, such as the second waist region 122, in the longitudinal and lateral directions. One example of the manually formed tuck flap is shown in Fig. 4B. As previously mentioned, the manually formed tuck flap may have shapes other than what are illustrated in the figures. It is also to be appreciated that a chassis having third and fourth ears 146, 148 such as shown in Fig. 4B may include a pre-formed tuck flap that may or may not be stretchable.

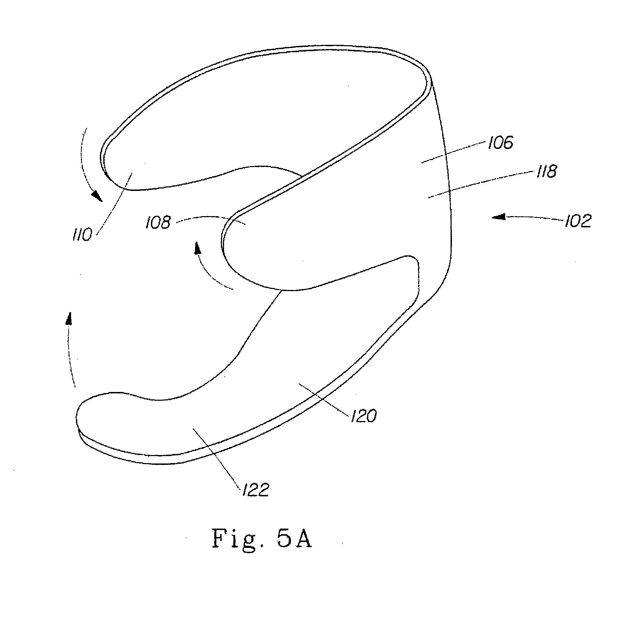

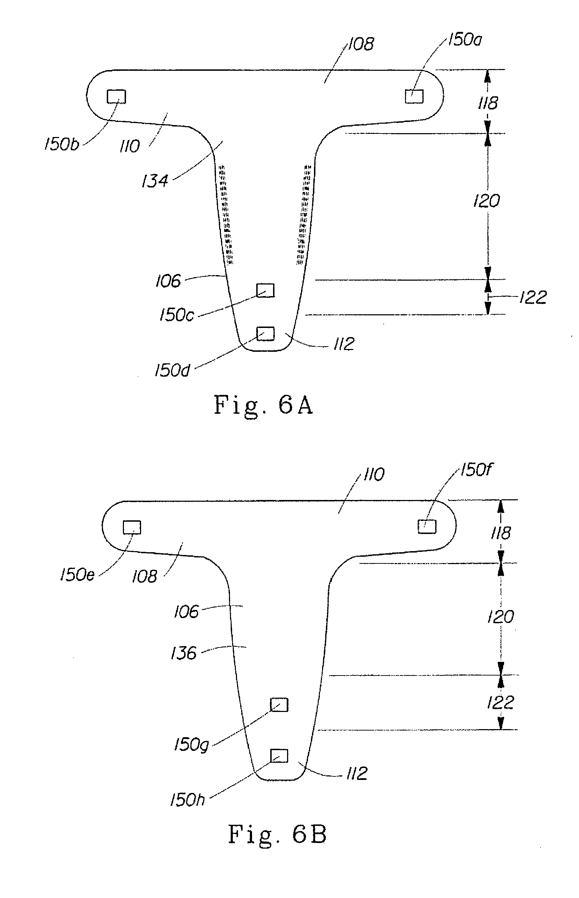

As discussed in more detail below, the diaper 102 may be placed on a wearer's body by positioning the first waist region 118 of the diaper adjacent to a rear waist region of a wearer's torso. The second waist region 122 may then be passed between a wearer's legs to position the crotch region 120 of the chassis 106 along a wearer's crotch area. The first and second ears 108, 110 are also folded to curve and extend generally around the longitudinal axis 114 of Fig. 1 and form a belt around a wearer's waist. Fig. 5A illustrates general directions in which the first ear 108, second ear 110, crotch region 120, and second waist region 122 may be folded when placing the diaper 102 on a wearer's body. The ears 108, 110 may be wrapped around the waist area of the wearer's lower torso and engage a portion of the second waist region 122 and/or tuck flap 112 along the wearer's front waist region to secure the diaper on the wearer. When the diaper 102 is placed on a wearer's body, portions of the longitudinally extending side edges 126, 128 define leg openings that encircle the wearer's thighs to help prevent leakage of bodily exudates therearound. As discussed in more detail below with reference to Figs. 6A-7B and others, the absorbent article 100 may include one or more fastener or fastening elements 150 adapted to connect the ears with each other and/or other portions of the chassis, such as the second waist region and/or the tuck flap. Also, as described below, the ears, second waist region, and tuck flap can engage each other in various ways to secure the diaper on a wearer's body.

The following provides a description of some of the various structural variations that may be included with any of the presently disclosed diaper and chassis embodiments, such as described above with reference to Figs. 1-5A.

As previously mentioned, the chassis 106 of the diaper 102 may include the backsheet 142, shown for example, in Fig. 1. In some embodiments, the backsheet is configured to prevent exudates absorbed and contained within the chassis from soiling articles that may contact the diaper, such as bedsheets and undergarments. Some embodiments of the backsheet may be fluid permeable, while other embodiments may be impervious to liquids (e.g., urine) and comprises a thin plastic film. In some embodiments, the plastic film includes a thermoplastic film having a thickness of about 0.012 mm (0.5 mil) to about 0.051 mm (2.0 mils). Some backsheet films may include those manufactured by Tredegar Industries Inc. of Terre Haute, Ind. and sold under the trade names X15306, X10962, and X10964. Other backsheet materials may include breathable materials that permit vapors to escape from the diaper while still preventing exudates from passing through the backsheet. Exemplary breathable materials may include materials such as woven webs, nonwoven webs, composite materials such as film-coated nonwoven webs, and microporous films such as manufactured by Mitsui Toatsu Co., of Japan under the designation ESPOIR NO and by EXXON Chemical Co., of Bay City, Tex., under the designation EXXAIRE. Suitable breathable composite materials comprising polymer blends are available from Clopay Corporation, Cincinnati, Ohio under the name HYTREL blend P18-3097. Such breathable composite materials are described in greater detail in PCT Application No. WO 95/16746, published on Jun. 22, 1995 in the name of E. I. DuPont and U.S. Pat. No. 5,865,823, issued on Feb. 2, 1999 to Curro, both of which are hereby incorporated by reference herein. Other breathable backsheets including nonwoven webs and apertured formed films are described in U.S. Pat. No. 5,571,096 issued to Dobrin et al. on Nov. 5, 1996; and U.S. Pat. No. 6,573, 423 issued to Herrlein et al. on June 3, 2003, which are all hereby incorporated by reference herein.

The backsheet 142, or any portion thereof, may be stretchable in one or more directions. In one embodiment, the backsheet may comprise a structural elastic-like film ("SELF") web. Embodiments of SELF webs are more completely described in U.S. Pat. No. 5,518,801, entitled "Web Materials Exhibiting Elastic-L.ike Behavior," which issued to Chappell et al. on May 21, 1996, U.S. Pat. No. 5,723,087, entitled "Web Materials Exhibiting Elastic-Like Behavior," which issued to Chappell et al. on Mar. 3, 1998; U.S. Pat. No. 5,691,035, entitled "Web Materials Exhibiting Elastic-Like Behavior," which issued to Chappell et al. on Nov. 25, 1997; U.S. Pat. No. 5,891,544, entitled "Web Materials Exhibiting Elastic-Like Behavior," which issued to Chappell et al. on Apr. 6, 1999; U.S. Pat. No. 5,916,663, entitled "Web Materials Exhibiting Elastic-Like Behavior," which issued to Chappell et al. on Jun. 29, 1999; and U.S. Pat. No. 6,027,483, entitled "Web Materials Exhibiting Elastic-Like Behavior," which issued to Chappell et al. on Fe. 22, 2000, which are all hereby incorporated by reference herein. In some embodiments, the backsheet may comprise elastomeric films, foams, strands, nonwovens, or combinations of these or other suitable materials with nonwovens or synthetic films. Additional embodiments include backsheets that comprise a stretch nonwoven material; an elastomeric film in combination with an extensible nonwoven; an elastomeric nonwoven in combination with an extensible film; and/or combinations thereof. Details on such backsheet embodiments are more completely described in U.S. non-provisional patent application entitled "Biaxially Stretchable Outer Cover for an Absorbent Article," filed on Nov. 15, 2006 with Express Mail No. EV916939625US and further identified by attorney docket number 10643; U.S. non-provisional patent application entitled "Disposable Wearable Articles with Anchoring Systems," filed on Nov. 15, 2006 with Express Mail No. EV916939648US and further identified by attorney docket number 10628Q; and U.S. non-provisional patent application entitled "Absorbent Article having an Anchored Core Assembly," tiled on Nov. 15, 2006 with Express Mail No. EV916939634US and further identified by attorney docket number 10432MQ, which are all hereby incorporated by reference herein.

The backsheet 142 may be joined with the topsheet 140, the absorbent core 144, and/or other elements of the diaper 102 in various ways. For example, the backsheet may be connected with a uniform continuous layer of adhesive, a patterned layer of adhesive, or an array of separate lines, spirals, or spots of adhesive. One embodiment utilizes an open pattern network of filaments of adhesive as disclosed in U.S. Pat. No. 4,573,986, entitled "Disposable Waste-Containment Garment," which issued to Minetola et al. on Mar. 4, 1986, which is hereby incorporated by reference herein. Other embodiments utilize several lines of adhesive filaments which are swirled into a spiral pattern, as is illustrated by the apparatus and methods shown in U.S. Pat. No. 3,911,173, issued to Sprague, Jr. on Oct. 7, 1975; U.S. Pat. No. 4,785,996, issued to Ziecker, et al. on Nov. 22, 1988; and U.S. Pat. No. 4,842,666 issued to Werenicz on Jun. 27, 1989, which are all hereby incorporated by reference herein. Adhesives may includes those manufactured by H. B. Fuller Company of St. Paul, Minn. and marketed as HL-1620 and HL-1358-XZP. In some embodiments, the backsheet is connected with heat bonds, pressure bonds, ultrasonic bonds, dynamic mechanical bonds, or any other suitable attachment means or a combination thereof.

The topsheet 140 may be joined to the backsheet 142, the absorbent core 144, and/or other elements of the diaper 102 in various ways. For example, the topsheet 140 may be connected in ways described above with respect to joining the backsheet 142 to other elements of the diaper 102. In one embodiment, the topsheet 140 and the backsheet 142 are joined directly to each other along the outer edge of the chassis. In another embodiment, the topsheet and the backsheet are joined directly to each other in some locations and are indirectly joined together in other locations. Other topsheet and backsheet connection configurations are described in more detail in U.S. provisional patent application number 60/811,700, entitled "Absorbent Article Having a Multifunctional Containment Member," filed on June 7, 2006, which is hereby incorporated by reference herein.

The topsheet 140 may be constructed to be compliant, soft feeling, and non-irritating to the wearer's skin. Further, all or at least a portion of the topsheet 140 may be liquid pervious, permitting liquid to readily penetrate therethrough. As such, the topsheet may be manufactured from a wide range of materials, such as porous foams; reticulated foams; apertured nonwovens or plastic films; or woven or nonwoven webs of natural fibers (e.g., wood or cotton fibers), synthetic fibers (e.g., polyester or polypropylene fibers), or a combination of natural and synthetic fibers. If the absorbent assemblies include fibers, the fibers may be spunbonded, carded, wet-laid, meltblown, hydroentangled, or otherwise processed as is known in the art. One example of a topsheet including a web of staple length polypropylene fibers is manufactured by Veratec, Inc., a Division of International Paper Company, of Walpole, Mass. under the designation P-8.

Examples of formed film topsheets are described in U.S. Pat. No. 3,929,135, entitled "Absorptive Structures Having Tapered Capillaries," which issued to Thompson on Dec. 30, 1975; U.S. Pat. No. 4,324,246, entitled "Disposable Absorbent Article Having A Stain Resistant Topsheet," which issued to Mullane, et al. on Apr. 13, 1982; U.S. Pat. No. 4,342,314, entitled "Resilient Plastic Web Exhibiting Fiber-Like Properties," which issued to Radel, et al. on Aug. 3, 1982; U.S. Pat. No. 4,463,045, entitled "Macroscopically Expanded Three-Dimensional Plastic Web Exhibiting Non-Glossy Visible Surface and Cloth-Like Tactile Impression," which issued to Ahr, et al. on Jul. 31, 1984; and U.S. Pat. No. 5,006,394, entitled "Multilayer Polymeric Film," which issued to Baird on Apr. 9, 1991, all of which are hereby incorporated by reference herein. Other topsheets may be made in accordance with U.S. Pat. Nos. 4,609,518 and 4,629,643, which issued to Curro et al. on Sep. 2, 1986, and Dec. 16, 1986, respectively, both of which are hereby incorporated by reference herein. Such formed films are available from The Procter & Gamble Company of Cincinnati, Ohio as "DRI-WEAVE" and from Tredegar Corporation of Terre Haute, Ind. as "CLIFF-T."

In some embodiments, the topsheet 140 is made of a hydrophobic material or is treated to be hydrophobic in order to isolate the wearer's skin from liquids contained in the absorbent core. If the topsheet is made of a hydrophobic material, at least the upper surface of the topsheet may be treated to be hydrophilic so that liquids will transfer through the topsheet more rapidly. This diminishes the likelihood that body exudates will flow off the topsheet rather than being drawn through the topsheet and being absorbed by the absorbent core. The topsheet can be rendered hydrophilic by treating it with a surfactant or by incorporating a surfactant into the topsheet. Suitable methods for treating the topsheet with a surfactant include spraying the topsheet material with the surfactant and immersing the material into the surfactant. A more detailed discussion of such a treatment and hydrophilicity is contained in U.S. Pat. No. 4,988,344, entitled "Absorbent Articles with Multiple Layer Absorbent Layers," which issued to Reising, et al. on Jan. 29, 1991, and U.S. Pat. No. 4,988,345, entitled "Absorbent Articles with Rapid Acquiring Absorbent Cores," which issued to Reising on Jan. 29, 1991, all of which are hereby incorporated by reference herein. A more detailed discussion of some methods for incorporating surfactant in the topsheet can be found in U.S. Statutory Invention Registration No. H1670, which was published on Jul. 1, 1997, in the names of Aziz et al., all of which are hereby incorporated by reference herein.

In some embodiments, the topsheet 140 may include an apertured web or film that is hydrophobic. This may be accomplished eliminating the hydrophilizing treatment step from the production process and/or applying a hydrophobic treatment to the topsheet, such as a polytetrafluoroethylene compound like SCOTCHGUARD or a hydrophobic lotion composition, as described below. In such embodiments, the apertures may be large enough to allow the penetration of aqueous fluids like urine without significant resistance. A more detailed discussion of various apertured topsheets can be found in U.S. Pat. No. 5,342,338, entitled "Disposable Absorbent Article for Low-Viscosity Fecal Material," which issued to Roe on Aug. 30, 1994; U.S. Pat. No. 5,941,864, entitled "Disposable Absorbent Article having Improved Fecal Storage," which issued to Roe on Aug. 24, 1999; U.S. Pat. No. 6,010,491, entitled "Viscous Fluid Bodily Waste Management Article," which issued to Roe et al. on Jan. 4, 2000; and U.S. Pat. No. 6,414,215, entitled "Disposable Absorbent Article having Capacity to Store Low-Viscosity Fecal Material," which issued to Roe on July 2, 20002, all of which are hereby incorporated by referenced herein.

Any portion of the topsheet 140 may be coated with a lotion, such as topsheets described in U.S. Pat. No. 5,607,760, entitled "Disposable Absorbent Article Having A Lotioned Topsheet Containing an Emollient and a Polyol Polyester Immobilizing Agent," which issued to Roe on Mar. 4, 1997; U.S. Pat. No. 5,609,587, entitled "Diaper Having A Lotion Topsheet Comprising A Liquid Polyol Polyester Emollient And An Immobilizing Agent," which issued to Roe on Mar. 11, 1997; U.S. Pat. No. 5,635,191, entitled "Diaper Having A Lotioned Topsheet Containing A Polysiloxane Emollient," which issued to Roe et al. on Jun. 3, 1997; U.S. Pat. No. 5,643,588, entitled "Diaper Having A Lotioned Topsheet," which issued to Roe et al. on Jul. 1, 1997; and U.S. Pat. No. 6,498,284, entitled "Disposable Absorbent Article with a Skin Care Composition on an Apertured Top Sheet," which issued to Roe on Dec. 24, 2002, all of which are hereby incorporated by reference herein. The lotion may function alone or in combination with another agent as the hydrophobizing treatment described above. The topsheet may also include or be treated with antibacterial agents, some examples of which are disclosed in PCT Publication No. WO 95/24173 entitled "Absorbent Articles Containing Antibacterial Agents in the Topsheet For Odor Control," which was published on Sep. 14, 1995, in the name of Theresa Johnson, which is hereby incorporated by reference herein. Further, the topsheet, the backsheet, or any portion of the topsheet or backsheet may be embossed and/or matte finished to provide a more cloth like appearance.

Embodiments of the absorbent article may also include pockets for receiving and containing waste, spacers which provide voids for waste, barriers for limiting the movement of waste in the article, compartments or voids which accept and contain waste materials deposited in the diaper, and the like, or any combinations thereof. Examples of pockets and spacers for use in absorbent products are described in U.S. Pat. No. 5,514,121 issued to Roe et al. on May 7, 1996, entitled "Diaper Having Expulsive Spacer"; U.S. Pat. No. 5,171,236 issued to Dreier et al on Dec. 15, 1992, entitled "Disposable Absorbent Article Having Core Spacers"; U.S. Pat. No. 5,397,318 issued to Dreier on Mar. 14, 1995, entitled "Absorbent Article Having A Pocket Cuff"; U.S. Pat. No. 5,540,671 issued to Dreier on Jul. 30, 1996, entitled "Absorbent Article Having A Pocket Cuff With An Apex"; and PCT Application WO 93/25172 published Dec. 3, 1993, entitled "Spacers For Use In Hygienic Absorbent Articles And Disposable Absorbent Articles Having Such Spacer"; and U.S. Pat. No. 5,306,266, entitled "Flexible Spacers For Use In Disposable Absorbent Articles", issued to Freeland on Apr. 26, 1994, which are all hereby incorporated by reference herein. Examples of compartments or voids are disclosed in U.S. Pat. No. 4,968,312, entitled "Disposable Fecal Compartmenting Diaper", issued to Khan on Nov. 6, 1990; U.S. Pat. No. 4,990,147, entitled "Absorbent Article With Elastic Liner For Waste Material Isolation", issued to Freeland on Feb. 5, 1991; U.S. Pat. No. 5,062,840, entitled "Disposable Diapers", issued to Holt et al on Nov. 5, 1991; U.S. Pat. No. 6,482,191 entitled "Elasticated Topsheet with an Elongate Slit Opening," issued to Roe et al. on Nov. 19, 2002; and U.S. Pat. No. 5,269,755 entitled "Trisection Topsheets For Disposable Absorbent Articles And Disposable Absorbent Articles Having Such Trisection Topsheets", issued to Freeland et al. on Dec. 14, 1993, which are all hereby incorporated by reference herein. Examples of suitable transverse barriers are described in U.S. Pat. No. 5,554,142 entitled "Absorbent Article Having Multiple Effective Height Transverse Partition" issued Sep. 10, 1996 in the name of Dreier et al.; PCT Patent WO 94/14395 entitled "Absorbent Article Having An Upstanding Transverse Partition" published Jul. 7, 1994 in the name of Freeland, et al., and U.S. Pat No. 5,653,703 Absorbent Article Having Angular Upstanding Transverse Partition, issued Aug. 5, 1997 to Roe, et al., which are all hereby incorporated by reference herein. All of the above-cited references are hereby incorporated by reference herein. In addition to or in place of the voids, pockets and barriers, described above, embodiments of the absorbent article may also include a waste management element capable of effectively and efficiently accepting, storing and/or immobilizing viscous fluid bodily waste, such as runny feces, such as described in U.S. Pat. No. 6,010,491 issued to Roe et al. on Jan. 4, 2000, which is hereby incorporated by reference herein.

The absorbent core 144 may include absorbent material that is generally compressible, conformable, non-irritating to the wearer's skin, and capable of absorbing and retaining liquids such as urine and other body exudates. The absorbent core 144 can also be manufactured in a wide variety of sizes and shapes (e.g., rectangular, hourglass, T-shaped, asymmetric, etc.). The absorbent core 144 may also include a wide variety of liquid-absorbent materials commonly used in disposable diapers and other absorbent articles. In one example, the absorbent core includes comminuted wood pulp, which is generally referred to as airfelt. Examples of other absorbent materials include creped cellulose wadding; meltblown polymers, including coform; chemically stiffened, modified or cross-linked cellulosic fibers; tissue, including tissue wraps and tissue laminates; absorbent foams; absorbent sponges; superabsorbent polymers; absorbent gelling materials; or any other known absorbent material or combinations of materials.

It is to be appreciated that the configuration and construction of the absorbent core 144 may be varied (e.g., the absorbent core(s) or other absorbent structure(s) may have varying caliper zones, a hydrophilic gradient, a superabsorbent gradient, or lower average density and lower average basis weight acquisition zones; or may comprise one or more layers or structures).

Exemplary absorbent structures are described in U.S. Pat. No. 4,610,678, entitled "High-Density Absorbent Structures," which issued to Weisman et al. on Sep. 9, 1986; U.S. Pat. No. 4,673,402, entitled "Absorbent Articles With Dual-Layered Cores," which issued to Weisman et al. on Jun. 16, 1987; U.S. Pat. No. 4,834,735, entitled "High Density Absorbent Members Having Lower Density and Lower Basis Weight Acquisition Zones," which issued to Alemany et al. on May 30, 1989; U.S. Pat. No. 4,888,231, entitled "Absorbent Core Having A Dusting Layer," which issued to Angstadt on Dec. 19, 1989; U.S. Pat. No. 5,137,537, entitled "Absorbent Structure Containing Individualized, Polycarboxylic Acid Crosslinked Wood Pulp Cellulose Fibers," which issued to Herron et al. on Aug. 11, 1992; U.S. Pat. No. 5,147,345, entitled "High Efficiency Absorbent Articles For Incontinence Management," which issued to Young et al. on Sep. 15, 1992; U.S. Pat. No. 5,342,338, entitled "Disposable Absorbent Article For Low-Viscosity Fecal Material," issued to Roe on Aug. 30, 1994; U.S. Pat. No. 5,260,345, entitled "Absorbent Foam Materials For Aqueous Body Fluids and Absorbent Articles Containing Such Materials," which issued to DesMarais et al. on Nov. 9, 1993; U.S. Pat. No. 5,387,207, entitled "Thin-Until-Wet Absorbent Foam Materials For Aqueous Body Fluids And Process For Making Same," which issued to Dyer et al. on Feb. 7, 1995; and U.S. Pat. No. 5,650,222, entitled "Absorbent Foam Materials For Aqueous Fluids Made From high Internal Phase Emulsions Having Very High Water-To-Oil Ratios," which issued to DesMarais et al. on Jul. 22, 1997, all of which are hereby incorporated by reference herein.

The absorbent core 144 may also have a multiple layered construction. A more detailed discussion of various types of multi-layered absorbent cores can be found in U.S. Pat. No. 5,669,894, entitled "Absorbent Members for Body Fluids having Good Wet Integrity and Relatively High Concentrations of Hydrogel-forming Absorbent Polymer," issued to Goldman et al. on Sept. 23, 1997; U.S. Pat. No. 6,441,266, entitled "Absorbent Members for Body Fluids using Hydrogel-forming Absorbent Polymer," issued to Dyer et al. on Aug. 26, 2002; U.S. Pat. No. 5,562,646, entitled "Absorbent Members for Body Fluids having Good Wet Integrity and Relatively High Concentrations of Hydrogel-forming Absorbent Polymer having High Porosity," issued to Goldman et al. on Oct. 10, 1996; European Pat. No. EP0565606B1, published on Mar. 8, 1995; U.S. Pat. Publication No. 2004/0162536A1 published Aug. 19, 2004; U.S. Pat. Publication No. 2004/0167486A1 published on Aug. 26, 2004; and PCT Publication No. WO 2006/015141 published on Feb. 9, 2006, which are all hereby incorporated by reference herein. In some embodiments, the absorbent article includes an absorbent core that is stretchable. In such a configuration, the absorbent core may be adapted to extend along with other materials of the chassis in longitudinal and/or lateral directions. The absorbent core can also be connected with the other components of the chassis various ways. For example, the diaper may include a "floating core" configuration or a "bucket" configuration wherein the diaper includes an anchoring system that can be configured to collect forces tending to move the article on the wearer. Such an anchoring system can also be configured to anchor itself to a body of a wearer by contacting various parts of the body. In this way, the anchoring system can balance the collected moving forces with holding forces obtained from the anchoring. By balancing the collected moving forces with the obtained holding forces, the anchoring system can at least assist in holding the disposable wearable absorbent article in place on a wearer. A more detailed discussion of various floating and/or bucket core configurations can be found in U.S. provisional patent application number 60/811,700, entitled "Absorbent Article Having a Multifunctional Containment Member," filed on June 7, 2006; U.S. non-provisional patent application entitled "Disposable Wearable Articles with Anchoring Systems," filed on Nov. 15, 2006 with Express Mail No. EV916939648US and further identified by attorney docket number 10628Q; and U.S. non-provisional patent application entitled "Absorbent Article having an Anchored Core Assembly," filed on Nov. 15, 2006 with Express Mail No. EV916939634US and further identified by attorney docket number 10432MQ, which are all hereby incorporated by reference herein.

The diaper 102 may also include at least one elastic waist feature 152, shown for example in Fig. 1, which may provide improved fit and waste containment. The elastic waist feature 152 may be configured to elastically expand and contract to dynamically fit the wearer's waist. The elastic waist feature 152 may extend at least longitudinally outwardly from the absorbent core 144 and generally form at least a portion of the first outer edge 130 of the diaper 102. In addition, the elastic waist feature may extend laterally to include the first and second ears 108, 110. While the elastic waist feature 152 or any constituent elements thereof may comprise one or more separate elements affixed to the diaper, the elastic waist feature may be constructed as an extension of other elements of the diaper, such as the backsheet 142, the topsheet 140, or both the backsheet and the topsheet. In addition, the elastic waist feature 152 may be disposed on the outer, garment-facing surface 136 of the chassis 106; the inner, wearer or body-facing surface 134; or between the inner and outer facing surfaces.

The elastic waist feature 152 may be constructed in a number of different configurations including those described in U.S. Pat. No. 4,515,595, which issued to Kievit et al. on May 7, 1985; U.S. Pat. No. 4,710,189, which issued to Lasch on Dec. 1, 1987; U.S. Pat. No. 5,151,092, which issued to Buell on Sep. 9, 1992; and U.S. Pat. No. 5,221,274, which issued to Buell on Jun. 22, 1993, all of which are hereby incorporated by reference herein. Other waist configurations may include waistcap features such as those described in U.S. Pat. No. 5,026,364, which issued to Robertson on Jun. 25, 1991 and U.S. Pat. No. 4,816,025, which issued to Foreman on Mar. 28, 1989, both of which are hereby incorporated by reference herein.

Although the first and second ears 108, 110 shown in Fig. 1 as well as the third and fourth ears 146, 148 shown in Fig. 4A and others are illustrated as being integrally formed with the chassis 106, it is to be appreciated that other embodiments may include ears that are discrete elements connected with the chassis. In some embodiments, the ears are configured to be stretchable, and in some embodiments, it may be preferable to have elastically stretchable ears. As discussed in more detail below, the ears may also include one or more fastener or fastening elements 150 adapted to releasably connect with each other and/or other fastener elements on the chassis. A more detailed discussion of stretchable ears can be found in U.S. Pat. No. 4,857,067, entitled "Disposable Diaper Having Shirred Ears" issued to Wood, et al. on Aug. 15, 1989; U.S. Pat. No. 5,151,092 issued to Buell et al. on Sep. 29, 1992; U.S. Pat. No. 5,674,216 issued to Buell et al. on Oct. 7, 1997;, U.S. Pat. No. 6,677,258 issued to Carroll et al. on Jan. 13, 2004; U.S. Pat. No. 4,381,781 issued to Sciaraffa, et al. on May 3, 1983; U.S. Pat. No. 5,580,411 entitled "Zero Scrap Method For Manufacturing Side Panels For Absorbent Articles" issued to Nease, et al. on December 3, 1996; and U.S. Patent No. 6,004,306 entitled "Absorbent Article With Multi-Directional Extensible Side Panels" issued to Robles et al. on December 21, 1999, which are all hereby incorporated by reference herein. The ears may also include various geometries and arrangements of stretch zones or elements, such as discussed in U.S. Pat. Publication No. US2005/0215972A1 published on Sept. 29, 2005, and U.S. Pat. Publication No. US2005/0215973A1 published on Sept. 29, 2005, which are all hereby incorporated by reference herein.

As shown in Fig. 1, the diaper 102 may include leg cuffs 154 that may provide improved containment of liquids and other body exudates. In particular, elastic gasketing leg cuffs can provide a sealing effect around the wearer's thighs to prevent leakage. It is to be appreciated that when the diaper is worn, the leg cuffs may be placed in contact with the wearer's thighs, and the extent of that contact and contact pressure may be determined in part by the orientation of diaper on the body of the wearer and the orientation of the ears and/or tuck flap relative to the longitudinal axis. The leg cuffs 154 may be disposed in various ways on the diaper 102. For example, the leg cuffs 154 may be disposed on the outer, garment-facing surface 136 of the chassis 106; the inner, wearer or body-facing surface 134; or between the inner and outer facing surfaces. Leg cuffs 154 may also be referred to as leg bands, side flaps, barrier cuffs, or elastic cuffs. U.S. Pat. No. 3,860,003, which is hereby incorporated by reference herein, describes a disposable diaper that provides a contractible leg opening having a side flap and one or more elastic members to provide an elasticized leg cuff (a gasketing cuff). U.S. Pat. Nos. 4,808,178 and 4,909,803, issued to Aziz et al. on Feb. 28, 1989, and Mar. 20, 1990, respectively, which are both hereby incorporated by reference herein, describe disposable diapers having "stand-up" elasticized flaps (barrier cuffs) which improve the containment of the leg regions. U.S. Pat. Nos. 4,695,278 and 4,795,454, issued to Lawson on Sep. 22, 1987, and to Dragoo on Jan. 3, 1989, respectively, which are both hereby incorporated by reference herein, describe disposable diapers having dual cuffs, including gasketing cuffs and barrier cuffs. In some embodiments, it may be desirable to treat all or a portion of the leg cuffs with a lotion, as described above. In addition to leg cuffs, diaper can also include an elastic gasketing cuff with one or more elastic strands positioned outboard of the barrier cuff. To improve waste containment, the leg cuffs may be treated with a hydrophobic surface coating, such as described in U.S. Pat. Publication No. 20060189956A1, entitled "Hydrophobic Surface Coated Light-Weight Nonwoven Laminates for Use in Absorbent Articles," published on Aug. 24, 2006, which is hereby incorporated by reference herein.

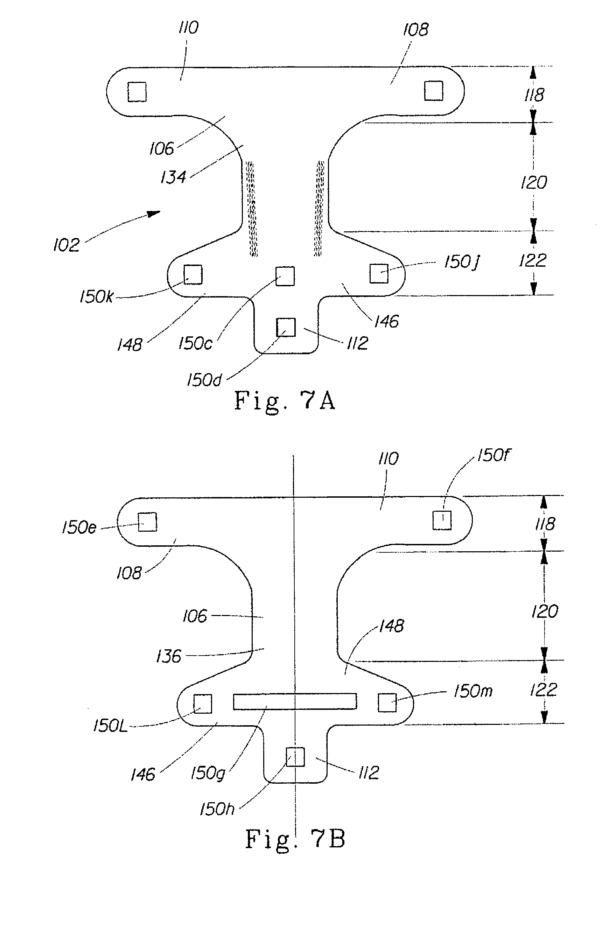

As previously mentioned, the diaper 102 may include fastener elements 150 in various locations to help secure the diaper in position on the wearer. Figs. 6A-7B illustrate various locations where fastener elements 150 may be located on the chassis 106. For example, Fig. 6A shows fastener elements in various locations on the outer, garment-facing surface of the chassis, and Fig. 6B shows fastener elements in various locations on the inner, body-facing surface of the chassis. With reference to Fig. 6A, the chassis 106 may include fastener elements 150a, 150b located on the inner surface 134 of the chassis adjacent the first ear 108 and the second ear 110, respectively. Additional fastener elements 150c, 150d may also be located on the inner surface 134 of the chassis adjacent the second waist region 122 and the tuck flap 112, respectively. With reference to Fig. 6B, the chassis 106 may include fastener elements 150e, 150f located on the outer surface 136 of the chassis adjacent the first ear 108 and the second ear 110, respectively. Additional fastener elements 150g, 150h may also be located on the outer surface 136 of the chassis adjacent the second waist region 122 and the tuck flap 112, respectively. Figs. 7A and 7B illustrate additional locations where fastener elements 150 may be located on the chassis 106 having third and fourth ears 146, 148. As shown in Fig. 7A, the chassis 106 may include fastener elements 150j, 150k located on the inner surface 134 of the chassis adjacent the third ear 146 and the fourth ear 148, respectively. As shown in Fig. 7B, the chassis 106 may include fastener elements 1501, 150m located on the outer surface 136 of the chassis adjacent the third ear 146 and the fourth ear 148, respectively.

It is to be appreciated that Figs. 6A-7B illustrate examples of fastener element locations, and therefore, may include fewer or larger numbers of fastener elements than those illustrated and may also define various shapes and sizes. For instance, multiple fastener elements 150 may be located on the inner and/or outer surfaces of the chassis 106 adjacent the tuck flap 112 to releasably connect the tuck flap with one or more regions of the chassis. It should also be appreciated that the fastening elements need not be adapted to connect with corresponding fastening elements on the chassis providing caregivers with increased flexibility in the application of the article to a wearer. In other words, the fastening elements can be adapted to connect directly with the chassis material as opposed to another fastening element. In addition, the fastening elements can be sized to cover large portions of various areas or regions on the chassis. For example, the entire area defined by the tuck flap or a vast majority thereof may be covered with fasteners, such as microhooks, adapted to connect with corresponding loops covering the entire area of the ears or a vast majority thereof, or vice versa. In yet another example, the vast majority or entirety of the tuck flap and/or ears may be covered with a cohesive. In some embodiments, a caregiver's ability to readily obtain a desired secure fit may be enhanced by increasing the size of the area of the diaper that is covered with fastening elements.

While the following description refers to Fig. 1A, it is to be appreciated that the following description is also applicable to other ear and tuck flap configurations, such as the ears 108, 110, 146, 148 and tuck flaps 112 shown in Figs. 6A-7B. As shown in Fig. 1A, the inner surface 134 and the outer surface 136 of each ear 108, 110 has an ear area 156 bounded by an ear perimeter 158. The ear perimeter 158 is defined by a laterally inboard ear edge 160, a laterally outboard ear edge 162, a longitudinally outboard ear edge 164, and a longitudinally inboard ear edge 166. The location of the laterally inboard ear edge 160 is defined by first locating points 168 on the side edges 126, 128 of the diaper in the crotch region 120 in a location that corresponds with the narrowest width between side edges 126, 128, and then drawing lines 170 through the points 168 parallel to the longitudinal axis 114 of the article. Also, as shown in Fig. 1A, each ear 108, 110 includes a distal ear portion 172 adjacent the laterally outboard ear edge 162; proximal ear portion 174 adjacent the laterally inboard ear edge 160; and a central ear portion 176 between the distal ear portion 172 and the proximal ear portion 174. In some embodiments, the distal, central, and proximal ear portions each have a lateral width defined by 1/3 the distance between the laterally inboard ear edge 160 and the laterally outboard ear edge 162.

In some embodiments, a substantial portion of at least one surface, such as the outer surface 136 or inner surface 134, of at least one ear 108, 110, 146, 148 is covered with fastening elements 150. For example, in some embodiments, at least one surface of at least one ear may have at least about 25% of the ear area covered with fastening elements, which are adapted to fasten with another portion of the article. In other embodiments, at least one surface of at least one ear may have at least about 50% of the ear area covered with fastening elements, which are adapted to fasten with another portion of the article. In yet other embodiments, at least one surface of at least one ear may have at least about 75% of the ear area covered with fastening elements, which are adapted to fasten with another portion of the article.

It is to be appreciated that in some embodiments, the at least one surface of the ear 108, 110, 146, 148 which is at least partially covered with fastening elements 150 is the body facing surface 134. In other embodiments, the surface of the ear which is at least partially covered with fastening elements is the garment facing surface 136. In yet other embodiments, at least some portion of both the body facing and the garment facing surfaces are at least partially covered with fastening elements. In some embodiments, the percentage of the ear area covered with fastening elements on the body facing surface may be the same or a different percentage of the ear area covered with fastening elements on the garment facing surface.

Some embodiments may include fastening elements disposed on either or both the body facing and garment facing surfaces 134, 136 that define a gradient of coverage. For example, the percentage of area of the distal ear portion 172 that is covered with fastening elements 150 may be different than the percentage of area of the central ear portion 176 or the proximal ear portion 174 that may be covered with fastening elements. In one example, percentage of area of the distal ear portion that is covered with fastening elements may be higher than the percentage of area of the proximal ear portion that is covered with fastening elements. In another example, percentage of area of the distal ear portion that is covered with fastening elements may be higher than the percentage of area of the central ear portion that is covered with fastening elements. In other examples, the percentage of area of the central portion that is covered with fastening elements may have be higher than the areas of the distal and/or proximal ear portions that are covered with fastening elements. It is to be appreciated that other configurations of gradients of coverage are also contemplated, such as for example, gradients extending in longitudinal directions, lateral directions, and/or combinations thereof.

As shown in Fig. 1A, the tuck flap 112 has a tuck flap area 178 bounded by a tuck flap perimeter 180, which is defined by portions of the side edges 126, 128 of the diaper extending between the second outer edge 132 and a tuck flap fold line 182. The tuck flap fold line 182 is a fold line extending across the tuck flap 112 when folded over the ears 108, 110, 146, 148. In some embodiments, at least 25% of the tuck flap area of at least one surface, either the outer surface 136 or the inner surface 134, of the tuck flap 112 is covered with fastening elements 150, which are adapted to connect with at least one other portion of the article 100. In other embodiments, at least about 50%, at least about 75%, or at least about 100% of the tuck flap area of at least one surface may be covered with fastening elements adapted to connect with at least one other portion of the article. In some embodiments, the garment facing surface (when viewed in a flat out, uncontracted, not folded configuration) of the tuck flap 112 is the surface at least partially covered with fastening elements. In some embodiments, the fastening elements on the tuck flap are adapted to connect with at least the fastening elements on the garment facing surface of at least one ear. In other embodiments, the fastening elements on the tuck flap are adapted to connect with at least the fastening elements on the wearer facing surface 134 of at least one ear 108, 110, 146, 148.

In some embodiments, fastening elements 150 may be disposed on the garment-facing surface 136 of the crotch 120 and/or waist regions 118, 122 of the article 100 inboard of the article perimeter. Such fastening elements may be in addition to or in place of any fastening elements on either an ear or a tuck flap surface. In such embodiments, an area of the article equivalent to about 50% of the ear and/or tuck flap area of the article may be covered by the fastening elements. In other embodiments, an area of the article equivalent to about 100% of the ear and/or tuck flap area of the article may be covered with fastening elements. In still other embodiments, an area of the article equivalent to about 200% of the ear and/or tuck flap area may be covered with fastening elements. In yet other embodiments, the area of the article comprising the fastening elements is sufficiently large to enable a caregiver to have significant flexibility as to where the ear or tuck flap is connected to the article, an ear, or tuck flap surface in order to accommodate the wide variation in baby sizes and proportions.

It is to be appreciated that various types of fastening elements 150 may be used with the diaper 102. In any of the preceding embodiments, the individual fastening elements may be relatively small. For example, the individual fastening elements may be sufficiently small as to be difficult to detect tactilely or visually. In some embodiments, the fastening elements include protrusions extending from a base surface. It is to be appreciated that the base surface may be defined by the inner surface 134 or outer surface 136 of the chassis 106, or a separate element attached thereto, such as a film. The protrusions may be adapted to entangle with fibrous structures capable of forming a connection with the protrusion. In other embodiments, the protrusions may be adapted to join via adhesion or other mechanism, such as interlocking, or combination of mechanisms to form a connection. Protrusions may be in the form of a hook or hook-like structure as known in the art as part of a mechanical fastening system or hook and loop fastening system. The protrusions may also be in the form of a rod, a knob, or other irregularly shaped object, a hairlike object, or other fibrous protrusion. Regardless of the form of a protrusion, a protrusion height can be defined as a maximum vertical distance the protrusion extends from the base surface to a highest point on the protrusion. In some embodiments, the protrusion height is less than about 0.5 mm. In other embodiments, the protrusion height is less than about 0.25 mm. In yet other embodiments, the protrusion height is less than about 0.1 mm. In still other embodiments, the protrusion height is less than about 0.01 mm. In articles including fastening elements having relatively short protrusion heights, the surface area covered by fastening elements may be greater than in other embodiments including fastening elements having relatively tall protrusion heights. Such a configuration may be advantageous for articles having fastening elements with relatively short protrusion heights that have either lower strength or may be individually less likely to capture a strand, such as from a loop landing zone or nonwoven attachment zone.

In one example, the fastening elements include hook & loop fasteners, such as those available from 3M or Velcro Industries. In other examples, the fastening elements include adhesives and/or tap tabs, while others are configured as a macrofastener or hook (e.g., a MACRO or "button-like" fastener). Some exemplary fastening elements and systems are disclosed in U.S. Pat. No. 3,848,594, entitled "Tape Fastening System for Disposable Diaper," which issued to Buell on Nov. 19, 1974; U.S. Pat. No. B1 4,662,875, entitled "Absorbent Article," which issued to Hirotsu et al. on May 5, 1987; U.S. Pat. No. 4,846,815, entitled "Disposable Diaper Having An Improved Fastening Device," which issued to Scripps on Jul. 11, 1989; U.S. Pat. No. 4,894,060, entitled "Disposable Diaper With Improved Hook Fastener Portion," which issued to Nestegard on Jan. 16, 1990; U.S. Pat. No. 4,946,527, entitled "Pressure-Sensitive Adhesive Fastener And Method of Making Same," which issued to Battrell on Aug. 7, 1990; and U.S. Pat. No. 5,151,092, issued to Buell on Sep. 29, 1992; and U.S. Pat. No. 5,221,274, which issued to Buell on Jun. 22, 1993, which are all hereby incorporated by reference herein. Additional examples of fasteners and/or fastening elements are discussed in U.S. Pat. Nos. 6,251,097 and 6,432,098; U.S. Patent Application Serial No. 11/240,943, entitled, "Anti-Pop Open Macrofasteners" filed on September 30, 2005; and U.S. Patent Application Serial No. 11/240,838, entitled, "A Fastening System Having Multiple Engagement Orientations", filed on September 30, 2005, which are all hereby incorporated by reference herein. Other fastening systems are described in more detail in U.S. Pat. No. 5,595,567 issued to King et al. on Jan. 21, 1997 and U.S. Pat. No. 5,624,427 issued to Bergman et al. on Apr. 29, 1997, both of which are entitled "Nonwoven Female Component For Refastenable Fastening Device." Yet other fastening systems are described in U.S. Pat. Nos. 5,735,840 and 5,928,212, both of which issued to Kline et al. and are entitled "Disposable Diaper With Integral Backsheet Landing Zone," which are both hereby incorporated by reference herein. Yet other fastening systems are described in publication WO 2005/065619 entitled "Gecko-like Fasteners for Disposable Article" and US Pat. No. 6,746,434 issued to Johnson et al. and entitled "Garment Having Integrally Formed Surface Protrusions," which are both hereby incorporated by reference herein. Alternative fastening systems include adhesive and cohesive fastening systems as known in the art, as well as combinations of adhesive, cohesive, and/or protrusion-based fasteners. An example of a suitable adhesive component may comprise discrete tape tabs, such as, for example, tape tabs available from the 3M Corporation of St. Paul, Minnesota, U.S.A. under the designation of XMF99121. An example of a cohesive component may comprise cohesive fastening patches. In some embodiments, the cohesive fastening patches may be formed of an inherently crystalline water-based synthetic elastomer to which a tackifying agent has been added to disrupt the polycrystalline structure and thereby render the elastomer cohesive. Exemplary synthetic cohesive products are available from Andover Coated Products, Incorporated, of Salisbury, Massachusetts, U.S.A. and are described in U.S. Patent No. 6,156,424. The fastening system may also provide a means for holding the article in a disposal configuration as disclosed in U.S. Pat. No. 4,963,140, which issued to Robertson et al. on Oct. 16, 1990, which is hereby incorporated by reference herein.

Another hook fastening material may include an array of prongs formed of thermoplastic material. Hot melt adhesive thermoplastics, in particular polyester and polyamide hot melt adhesives, may be particularly well suited for forming the prongs of the hook fastening material. The prongs, in some embodiments, can be manufactured using a modified gravure printing process by printing the thermoplastic material in its molten state onto a substrate in discrete units, severing the material in a manner that allows stretching of a portion of the thermoplastic material prior to severance, and allowing the stretched molten material to "freeze" resulting in prongs. This hook fastening material and methods and apparatus for making such a hook fastening material are more fully detailed in European Patent Application 0 381 087. In some embodiments, the hooks can be thermoplastically printed. Examples of suitable hook printing processes are described in U.S. Patent No. 5,540,673 and in WO 2004/082918.

As previously mentioned, fastening systems may also include a plurality of loops defining a receiving component. Loop fastening material and a method for making the same are described in U.S. Patent No. 5,380,313; U.S. Patent No. 5,569,233; U.S. Patent No. 5,407,439; U.S. Patent No. 5,542,942; U.S. Patent No. 5,669,900; U.S. Patent No. 5,318,555; U.S. Application Publication No. 2003/0077430; and WO 04/030763, which are all hereby incorporated by reference.

Based on the foregoing, it is to be appreciated that the chassis with the pre-formed tuck flap or manually formed tuck flap may also include various features provide for improved fit, comfort, and waste containment. Such features may include, as described above, stretchable ears, and elastic waist features that allow the diaper to conform to a wearer's particular anatomy. Various described absorbent core configurations may provide reduced dry thickness dimensions for better comfort and/or improved retention and distribution of bodily exudates. Other features described above include different ways in which the absorbent core is supported within the chassis that allow a chassis constructed from stretchable materials to maintain a snug fit on a wearer's body after the absorbent core has been insulted with bodily exudates. Other described features may include the use of nonwovens and/or apertured topsheets, and materials that are relatively soft, which may also be stretchable, to provide improved comfort and/or waste containment.

It is also to be appreciated that diapers 102 according the present disclosure may be constructed with various types of the previously described materials that allow the entire chassis 106 or portions of the chassis 106, such as the tuck flap 112, ears 108, 110, 146, 148, crotch region 120, and/or waist regions 118, 122 to stretch. It is to be appreciated that the entire chassis 106 or portions of the chassis can be configured to stretch in longitudinal directions, lateral directions, or both (i.e. biaxial stretch). In some embodiments, the chassis may include regions of longitudinal stretch, regions of lateral stretch, and/or regions of biaxial stretch. For example, in some embodiments, the entire length of the tuck flap 112 is adapted to stretch in longitudinal and/or lateral directions. In other embodiments, the distal end portion 124 of the tuck flap 112 is the only portion of the chassis 106 that is longitudinally and/or laterally stretchable. In yet other embodiments, central or proximal regions of the tuck flap 112 are the only portions of the chassis 106 that are longitudinally and/or laterally stretchable. In such example configurations, the tuck flap 112 or regions thereof may comprise a different material than that of the remainder of the chassis 106, may have been subjected to a different treatment (e.g. SELFing, mechanical ringrolling), or a combination thereof. References disclosing structural elastic-like film ("SELF") materials are discussed above. The tuck flap may also be constructed with a "zero strain" stretch laminate. Zero strain stretch laminates can be made by bonding an elastomer to a nonwoven while both are in an unstrained state. A more detailed discussion of zero strain laminates can be found in U.S. Pat. No. 5,156,793, entitled "Method for Incrementally Stretching Zero Strain Stretch Laminate Web in a Non-uniform Manner to Impart a Varying Degree of Elasticity Thereto," issued to Buell et al. on Oct. 20, 1992, which is hereby incorporated by reference herein. In another example, the tuck flap may be constructed with "live stretch," which may include stretching elastic and bonding the stretched elastic to a nonwoven. After bonding the stretched elastic is released causing it to contract, resulting in a "corrugated" nonwoven. A more detailed discussion of "live stretch" can be found in U.S. Pat. No. 4,720,415 to Vander Wielen, et al., issued Jan. 19, 1988 and U.S. Pat. No. 7,028,735 to Schneider et al. issued on April 18, 2006, which are hereby incorporated by reference herein.

As discussed above, the tuck flap 112 of the disclosed diapers 102 may be pre-formed or manually formed, or some combination thereof. In addition, embodiments of some pre-formed tuck flaps are stretchable, while other embodiments are not stretchable. Some pre-formed and manually formed tuck flaps 112 may be stretched in longitudinal and/or lateral directions before, during, and/or after placing the diaper on the wearer. Stretchable tuck flaps may provide some advantages over non-stretchable tuck flaps. For example, an individual placing the diaper on the wearer may extend the tuck flap to a length and/or width to provide a relatively better fit of the diaper on a wearer's particular anatomy. In addition to providing a better fit on the wearer, a chassis having a stretchable tuck flap may provide for reduced manufacturing costs over a chassis having a non-stretchable tuck flap. For example, embodiments having a non-stretchable, pre-formed tuck flap may be more expensive to produce than manually formed and/or stretchable tuck flaps due to the increased length of chassis material required for the pre-formed flap. In addition, stretchable components may allow the diaper to be manufactured with less material than might otherwise be required as well may reduce the need for special cutting and/or forming processes. Pre-formed tuck flaps that cannot stretch may also need to be cut to a longer dimension during manufacture and may be more complex to manufacture since tapered and/or curved ends of a pre-formed flap may need to be formed during the manufacturing process (e.g., via cutting), potentially leading to trim removal and web control issues. Conversely, stretchable flaps may provide pre-defined extended shaping of the tuck flap by controlling the degree to which various regions of the flap may extend under a given applied tension.

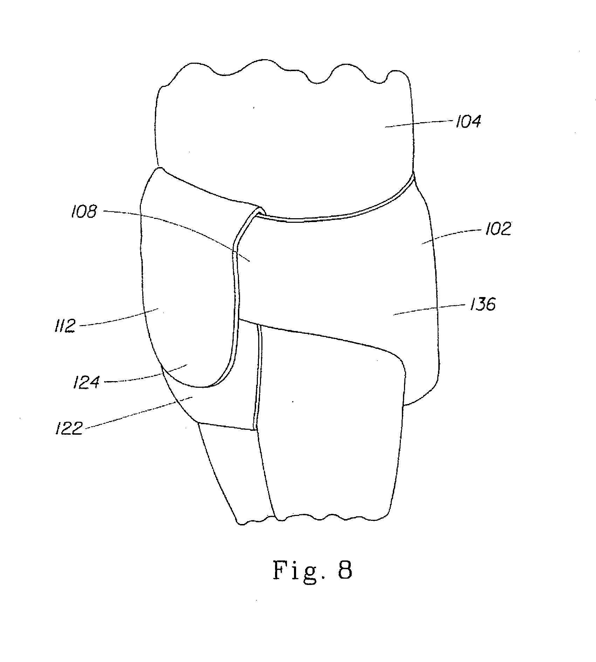

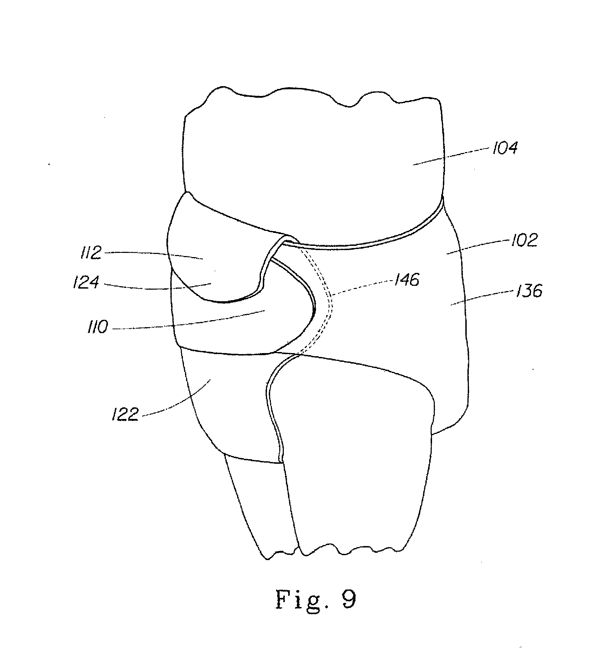

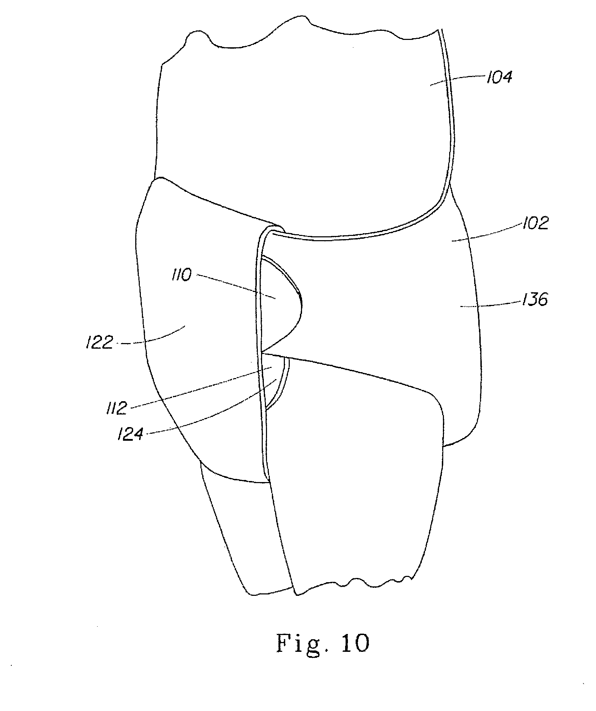

As previously mentioned, the ears 108, 110 (146, 148 if applicable); the second waist region 122, and the tuck flap 112 may be placed in various configurations to hold the diaper 102 on a wearer's body. The following provides a description of some of these various configurations as shown in Figs. 2 and 8-10. For example, the second waist region 122 may be disposed between the ears 108, 110 and the wearer's body with the tuck flap 112 folding over the ears and a portion of the second waist region, as shown in Figs. 2, 8, and 9. Depending on the particular arrangement of fastener elements 150, as described above with reference to Figs. 6A-7B, the ears 108, 110 may be releasably connected with each other as well as the second waist region 122 and/or the tuck flap 112. The distal end portion of the tuck flap may also be releasably connected with the second waist region 122 of the chassis 106. The ears 108, 110 may also be positioned in an overlapping arrangement across the second waist region 122, as shown in Fig. 2, or may extend over the second waist region 122 without overlapping, as shown in Fig. 8. Fig. 9 shows an example of the chassis 106 having third and fourth ears 146, 148 described above with reference to Figs. 4A and 4B with the second waist region 122 and third and fourth ears 146, 148 being disposed between the first and second ears 108, 110 and the wearer's body with the tuck flap 112 folding over the ears 108, 110. Again, depending on the particular arrangement of fastener elements 150, as described above with reference to Figs. 6A-7B, the ears 108, 110 may be releasably connected with the second waist region 122 and/or the tuck flap 112. The distal end portion of the tuck flap may also be releasably connected with the second waist region 122 of the chassis 106. In yet another example shown in Fig. 10, the ears 108, 110 may be disposed between the second waist region 122 and the tuck flap 112 with the tuck flap folding over the ears and adjacent to the wearer's body. With reference to Figs. 6A-7B and Fig. 10, the ears 108, 110 may be releasably connected with each other as well as the second waist region 122 and/or the tuck flap 112. The distal end portion of the tuck flap may also be releasably connected with the second waist region 122 of the chassis 106.

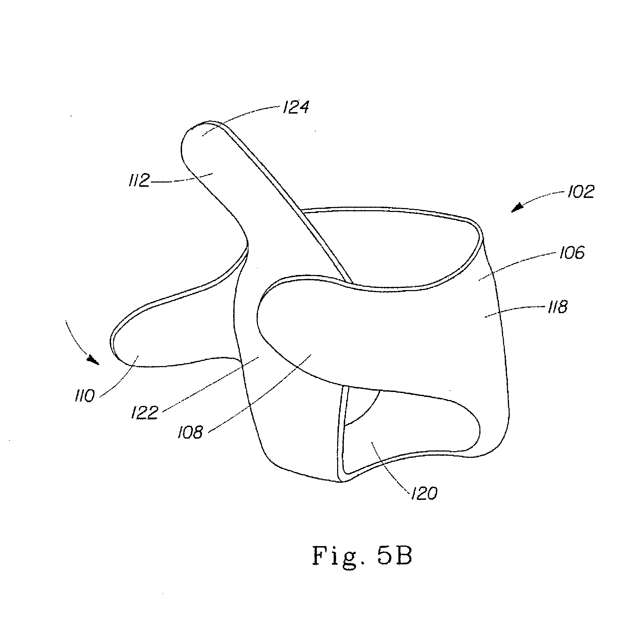

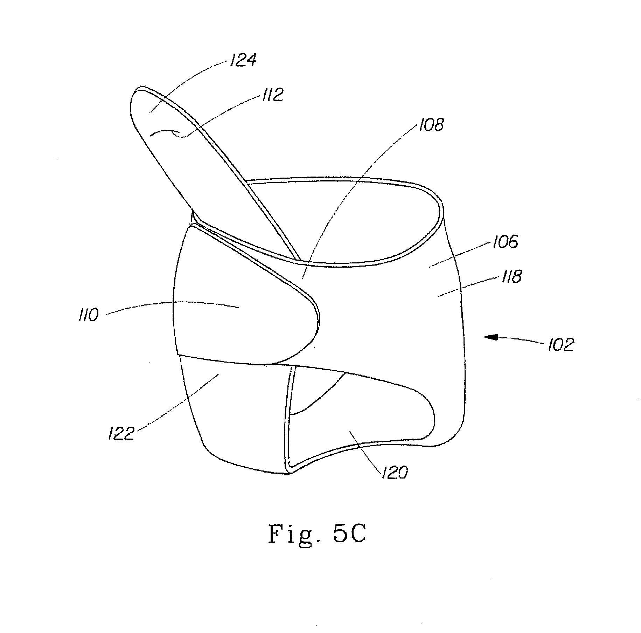

Based on the foregoing examples of wrap/tuck and folding configurations, it is to be appreciated that embodiments of the diaper 102 according to the present disclosure may be placed on wearer's body in various ways. For example, when folding the diaper 102 to be placed a wearer's body as shown in Figs. 2, by positioning the first waist region 118 of the chassis 106 adjacent a rear waist region of a wearer's torso. The second waist region 122 and crotch region 120 of the chassis 106 are then passed between the wearer's legs, placing the crotch region 120 along the wearer's crotch and the second waist region 122 adjacent the front waist region of the wearer's lower torso. The first and second ears 108, 110 are then wrapped around the waist of the wearer's lower torso to form a belt, as shown in Figs. 5A and 5B. The ears 108, 110 are then placed in an overlapping arrangement on the outer surface 136 of the chassis 106 adjacent the second waist region 122, as shown in Fig. 5C. It is to be appreciated that the order in which the aforementioned steps are performed may vary. For example, the ears 108, 110 could be first wrapped around the wearer's waist in an overlapping arrangement, and the tuck flap 112 then slid between the overlapped ears and the wearer's body.

The tuck flap 112 is then folded over the ears 108, 110 as shown in Fig. 2. As discussed above, the tuck flap may be pre-formed, stretchable, or manually formed. In addition, the tuck flap may also be stretched and/or formed before, during, and/or after placing the diaper on the wearer. Depending on the particular arrangement of fastener elements 150, which may vary as described above with reference to Figs. 6A and 6B, the ears 108, 110 may be releasably connected with each other as well as the second waist region 122 and/or the tuck flap 112. In addition, the tuck flap is shown to extend over the ears 108, 110 and downward to where the distal end portion 124 of the tuck flap 112 is adjacent the second waist region 122 of the chassis 108. As such, depending the arrangement of fastener elements 150, the distal end portion of the tuck flap may be releasably connected with the second waist region 122 of the chassis 106. It is to be appreciated that the tuck flap may be shorter than what is shown, and therefore, the distal end portion may not extend past the ears to the second waist region.

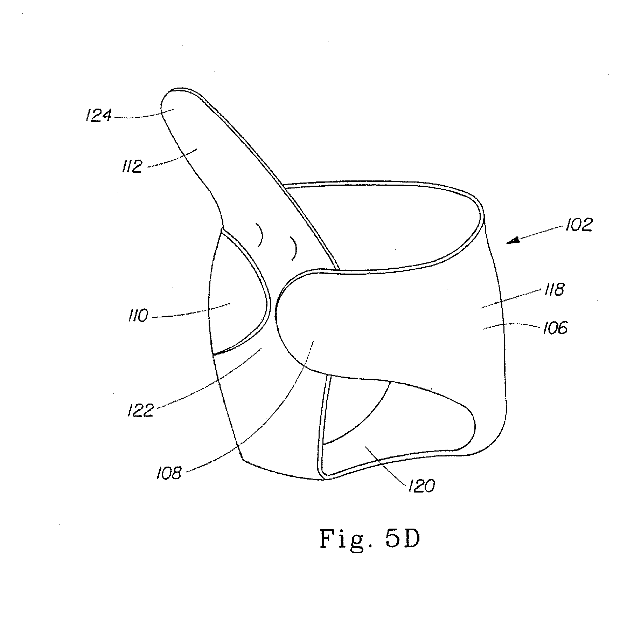

In a variation of the above example, the first and second ears 108, 110 are placed on the outer surface 136 of the chassis 106 adjacent the second waist region 122, as shown in Fig. 5D. The tuck flap 112 is then folded over the ears 108, 110 as shown in Fig. 8. Depending on the particular arrangement of fastener elements 150, which may vary as described above with reference to Figs. 6A and 6B, the ears 108, 110 may be releasably connected with the second waist region 122 and/or the tuck flap 112. In addition, the tuck flap is shown to extend over the ears 108, 110 and downward to where the distal end portion 124 of the tuck flap 112 is adjacent the second waist region 122 of the chassis 108. As such, depending the arrangement of fastener elements 150, the distal end portion of the tuck flap may be releasably connected with the second waist region 122 of the chassis 106. The tuck flap is also shown Fig. 8 to extend over the ears 108, 110 and downward to where the distal end portion 124 of the tuck flap 112 is adjacent the second waist region 122 of the chassis 108. As such, the distal end portion of the tuck flap may be releasably connected with the second waist region 122 of the chassis 106. It is to be appreciated that the tuck flap may be shorter than what is shown, and therefore, the distal end portion may not extend past the ears to the second waist region.

As previously mentioned, the tuck flap may be shorter than what is shown in some of the figures and may not extend past the ears to the second waist region. For example, Fig. 9 shows the chassis 106 described above with reference to Figs. 4A and 4B showing the tuck flap 112 as not extending past the ears 108, 110 to the second waist region. Fig. 9 also shows the third and fourth ears 146, 148 with the second waist region 122 and third and fourth ears 146, 148 being disposed between the first and second ears 108, 110 and the wearer's body. Depending on the particular arrangement of fastener elements 150, which may vary as described above with reference to Figs. 7A and 7B, the ears 108, 110 may be releasably connected with each other as well as the second waist region 122, the tuck flap 112, and/or ears 146, 148. It is to be appreciated that the tuck flap shown in Fig. 9 may also be longer than what is shown, and therefore, the distal end portion may extend past the ears to the second waist region.

Another variation of the above examples is shown in Fig. 10, wherein the ears 108, 110 are first wrapped around the waist of the wearer's lower torso to form a belt. The ears 108, 110 are then placed in an overlapping arrangement adjacent the wearer's front waist region. The second waist region 122 and crotch region 120 of the chassis 106 are passed between the wearer's legs, placing the crotch region 120 along the wearer's crotch and the second waist region 122 and adjacent the outer surfaces of the ears 108, 110. The tuck flap 112 is then folded over the ears 108, 110 between the inner surfaces of the ears and the wearer's body. Again, the tuck flap may be pre-formed, stretchable, or manually formed and may also be stretched and/or formed before, during, and/or after placing the diaper on the wearer. In addition, depending on the particular arrangement of fastener elements 150, which may vary as described above with reference to Figs. 6A and 6B, the ears 108, 110 may be releasably connected with each other as well as the second waist region 122 and/or the tuck flap 112. The tuck flap is also shown in Fig. 10 to extend over the ears 108, 110 and downward to where the distal end portion 124 of the tuck flap 112 is adjacent the second waist region 122 of the chassis 108. As such, the distal end portion of the tuck flap may be releasably connected with the second waist region 122 of the chassis 106. As discussed above, the tuck flap may be shorter than what is shown, and therefore, the distal end portion may not extend past the ears to the second waist region.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm".

All documents cited in the Detailed Description of the Invention are, in relevant part, incorporated herein by reference; the citation of any document is not to be construed as an admission that it is prior art with respect to the present invention. To the extent that any meaning or definition of a term in this written document conflicts with any meaning or definition of the term in a document incorporated by reference, the meaning or definition assigned to the term in this written document shall govern.

## Claims

1. A disposable absorbent article (100) adapted to be worn about a lower torso region of a wearer comprising:
a chassis (106) having an outer surface (136) and an inner surface (134) and including a first waist region (118), a second waist region (122), and a crotch region (120) disposed intermediate the first waist region (118) and the second waist region (122), the chassis (106) further including a first ear (108), a second ear (110), and a tuck flap (112), wherein the first and second ears (108, 110) extend in opposing lateral directions from the first waist region (118) and the tuck flap (112) extends in a longitudinal direction from the second waist region (122);
at least one fastener element (150) disposed on a first area of the chassis (106) and adapted to connect with a second area of the chassis (106); and
wherein at least one portion of the tuck flap (112) is stretchable in at least one direction; and
wherein the tuck flap (112) is stretchable in a longitudinal direction.

2. The disposable absorbent article of any of the preceding claims, wherein the first ear and the second ear (108, 110) are stretchable.

3. The disposable absorbent article of any of the preceding claims, wherein the first waist region (118) is stretchable.

4. The disposable absorbent article of any of the preceding claims, wherein the first waist region (118) is adapted to be positioned adjacent a wearer's rear waist region and the second waist region (122) is adapted to be positioned adjacent a wearer's front waist region.

5. The disposable absorbent article of any of the preceding claims, wherein the first ear (108) defines a first ear area on the inner surface and a first ear area on the outer surface;
wherein fastening elements are disposed on the first ear and are adapted to connect with a second area of the chassis; and
wherein at least 25% of either the first ear area on the inner surface or the first ear area on the outer surface is covered with the fastening elements, and preferably at least 50% of either the first ear area on the inner surface or the first ear area on the outer surface is covered with the fastening elements, and preferably 75% of either the first ear area on the inner surface or the first ear area on the outer surface is covered with the fastening elements.

6. The disposable absorbent article of claim 5, wherein the fastening elements comprise protrusions, wherein each of the protrusions define a protrusion height and wherein the protrusion height is less than 0.5 mm, 0.1 mm, or 0.01 mm.

7. The disposable absorbent article of any of the preceding claims, wherein the tuck flap defines a tuck flap area on the inner surface and a tuck flap area on the outer surface, and fastening elements are disposed on the tuck flap and adapted to connect with a second area of the chassis; and
wherein at least 25%, 50%, or 75% of either the tuck flap area on the inner surface or the tuck flap area on the outer surface is covered by the fastening elements, preferably the fastening elements comprise protrusions, wherein the protrusions define a protrusion height, and the protrusion height is less than 0.5 mm, 0.1 mm, or 0.01 mm.

8. The disposable absorbent article of any of the preceding claims, wherein the chassis defines a first chassis area on the inner surface and a second chassis area on the outer surface and fastening elements are disposed on the chassis and adapted to connect with either the tuck flap, the first ear, or the second ear; and wherein at least 25% either the first chassis area or the second chassis area is covered by the fastening elements, preferably the fastening elements comprise protrusions, wherein the protrusions define a protrusion height, wherein the protrusion height is less than about 0.5 mm.

## Patentansprüche

1. Einweg-Absorptionsartikel (100), der konzipiert ist, um einen Unterleibsbereich eines Trägers getragen zu werden, umfassend:
ein Gehäuse (106) mit einer Außenoberfläche (136) und einer Innenoberfläche (134) und einem ersten Taillenbereich (118), einem zweiten Taillenbereich (122) und einem Schrittbereich (120), der zwischen dem ersten Taillenbereich (118) und dem zweiten Taillenbereich (122) angeordnet ist, wobei das Gehäuse (106) ferner ein erstes Ohr (108), ein zweites Ohr (110) und eine Stecklasche (112) aufweist, wobei das erste und das zweite Ohr (108, 110) in entgegengesetzten lateralen Richtungen von dem ersten Taillenbereich (118) verlaufen und die Stecklasche (112) in einer Längsrichtung von dem zweiten Taillenbereich (122) verläuft,
mindestens ein Befestigungselement (150), das auf einem ersten Bereich des Gehäuses (106) angeordnet und konzipiert ist, mit einem zweiten Bereich des Gehäuses (106) verbunden zu werden, und
wobei mindestens ein Abschnitt der Stecklasche (112) in mindestens einer Richtung spannbar ist, und
wobei die Stecklasche (112) in eine Längsrichtung spannbar ist.

2. Einweg-Absorptionsartikel nach einem der vorstehenden Ansprüche, wobei das erste Ohr und das zweite Ohr (108, 110) spannbar sind.

3. Einweg-Absorptionsartikel nach einem der vorstehenden Ansprüche, wobei der erste Taillenbereich (118) spannbar ist.

4. Einweg-Absorptionsartikel nach einem der vorstehenden Ansprüche, wobei der erste Taillenbereich (118) konzipiert ist, benachbart eines hinteren Taillenbereichs eines Trägers angeordnet zu werden, und der zweite Taillenbereich (122) konzipiert ist, benachbart eines vorderen Taillenbereichs eines Trägers angeordnet zu werden.

5. Einweg-Absorptionsartikel nach einem der vorstehenden Ansprüche, wobei das erste Ohr (108) einen ersten Ohrbereich auf der Innenoberfläche und einen ersten Ohrbereich auf der Außenoberfläche definiert,
wobei die Befestigungselemente auf dem ersten Ohr angeordnet sind und konzipiert sind, mit einem zweiten Bereich des Gehäuses verbunden zu werden, und
wobei mindestens 25 % entweder des ersten Ohrbereichs auf der Innenoberfläche oder des ersten Ohrbereichs auf der Außenoberfläche mit den Befestigungselementen abgedeckt sind und vorzugsweise mindestens 50 % entweder des ersten Ohrbereichs auf der Innenoberfläche oder des ersten Ohrbereichs auf der Außenoberfläche mit den Befestigungselementen abgedeckt sind und vorzugsweise 75 % entweder des ersten Ohrbereichs auf der Innenoberfläche oder des ersten Ohrbereichs auf der Außenoberfläche mit den Befestigungselementen abgedeckt sind.

6. Einweg-Absorptionsartikel nach Anspruch 5, wobei die Befestigungselemente Vorsprünge umfassen, wobei jeder der Vorsprünge eine Vorsprunghöhe definiert und wobei die Vorsprunghöhe weniger als 0,5 mm, 0,1 mm oder 0,01 mm beträgt.

7. Einweg-Absorptionsartikel nach einem der vorstehenden Ansprüche, wobei die Stecklasche einen Stecklaschenbereich auf der Innenoberfläche und einen Stecklaschenbereich auf der Außenoberfläche definiert und Befestigungselemente auf der Stecklasche angeordnet und konzipiert sind, mit einem zweiten Bereich des Gehäuses verbunden zu werden, und
wobei mindestens 25 %, 50 % oder 75 % entweder des Stecklaschenbereichs auf der Innenoberfläche oder des Stecklaschenbereichs auf der Außenoberfläche durch die Befestigungselemente abgedeckt sind, wobei die Befestigungselemente vorzugsweise Vorsprünge umfassen, wobei die Vorsprünge eine Vorsprunghöhe definieren und die Vorsprunghöhe weniger als 0,5 mm, 0,1 mm oder 0,01 mm beträgt.

8. Einweg-Absorptionsartikel nach einem der vorstehenden Ansprüche, wobei das Gehäuse einen ersten Gehäusebereich auf der Innenoberfläche und einen zweiten Gehäusebereich auf der Außenoberfläche definiert und Befestigungselemente auf dem Gehäuse angeordnet und konzipiert sind, mit entweder der Stecklasche, dem ersten Ohr oder dem zweiten Ohr verbunden zu werden, und wobei mindestens 25 % entweder des ersten Gehäusebereichs oder des zweiten Gehäusebereichs von den Befestigungselementen abgedeckt sind, wobei die Befestigungselemente vorzugsweise Vorsprünge umfassen, wobei die Vorsprünge eine Vorsprunghöhe definieren, wobei die Vorsprunghöhe weniger als etwa 0,5 mm beträgt.

## Revendications

1. Article absorbant jetable (100) adapté pour être porté autour d'une région inférieure du tronc d'un porteur comprenant :
un châssis (106) ayant une surface externe (136) et une surface interne (134) et incluant une première région de ceinture (118), une deuxième région de ceinture (122), et une région d'entrejambe (120) disposée entre la première région de ceinture (118) et la deuxième région de ceinture (122), le châssis (106) incluant en outre une première patte (108), une deuxième patte (110), et un rabat rentrant (112), dans lequel les première et deuxième pattes (108, 110) s'étendent dans des directions latérales opposées à partir de la première région de ceinture (118) et le rabat rentrant (112) s'étend dans une direction longitudinale à partir de la deuxième région de ceinture (122) ;
au moins un élément de fermoir (150) disposé sur une première zone du châssis (106) et adapté pour se connecter à une deuxième zone du châssis (106) ; et
dans lequel au moins une partie du rabat rentrant (112) est étirable dans au moins une direction ; et
dans lequel le rabat rentrant (112) est étirable dans une direction longitudinale.

2. Article absorbant jetable selon l'une quelconque des revendications précédentes, dans lequel la première patte et la deuxième patte (108, 110) sont étirables.

3. Article absorbant jetable selon l'une quelconque des revendications précédentes, dans lequel la première région de ceinture (118) est étirable.

4. Article absorbant jetable selon l'une quelconque des revendications précédentes, dans lequel la première région de ceinture (118) est adaptée pour être positionnée adjacente à la région de ceinture arrière d'un porteur et la deuxième région de ceinture (122) est adapté pour être positionnée adjacente à la région de ceinture avant d'un porteur.

5. Article absorbant jetable selon l'une quelconque des revendications précédentes, dans lequel la première patte (108) définit une zone de première patte sur la surface interne et une zone de première patte sur la surface externe ;
dans lequel des éléments de fixation sont disposés sur la première patte et sont adaptés pour se connecter à une deuxième zone du châssis ; et
dans lequel au moins 25 % ou de la zone de première patte sur la surface interne ou de la zone de première patte sur la surface externe est couverte avec les éléments de fixation, et de préférence au moins 50 % ou de la zone de première patte sur la surface interne ou de la zone de première patte sur la surface externe est couverte avec les éléments de fixation, et de préférence 75 % ou de la zone de première patte sur la surface interne ou de la zone de première patte sur la surface externe est couverte avec les éléments de fixation.

6. Article absorbant jetable selon la revendication 5, dans lequel les éléments de fixation comprennent des parties saillantes, dans lequel chacune des parties saillantes définit une hauteur de partie saillante et dans lequel la hauteur de partie saillante est inférieure à 0,5 mm, 0,1 mm, ou 0,01 mm.

7. Article absorbant jetable selon l'une quelconque des revendications précédentes, dans lequel le rabat rentrant définit une zone de rabat rentrant sur la surface interne et une zone de rabat rentrant sur la surface externe, et les éléments de fixation sont disposés sur le rabat rentrant et adaptés pour se connecter à une deuxième zone du châssis ; et
dans lequel au moins 25 %, 50 %, ou 75 % ou de la zone de rabat rentrant sur la surface interne ou de la zone de rabat rentrant sur la surface externe est couverte par les éléments de fixation, de préférence les éléments de fixation comprennent des parties saillantes, les parties saillantes définissant une hauteur de partie saillante, et la hauteur de partie saillante étant inférieure à 0,5 mm, 0,1 mm, ou 0,01 mm.

8. Article absorbant jetable selon l'une quelconque des revendications précédentes, dans lequel le châssis définit une première zone de châssis sur la surface interne et une deuxième zone de châssis sur la surface externe et les éléments de fixation sont disposés sur le châssis et adaptés pour se connecter ou au rabat rentrant, ou à la première patte, ou à la deuxième patte ; et dans lequel au moins 25 % ou de la première zone de châssis ou de la deuxième zone de châssis est couverte par les éléments de fixation, de préférence les éléments de fixation comprennent des parties saillantes, les parties saillantes définissant une hauteur de partie saillante, la hauteur de partie saillante étant inférieure à environ 0,5 mm.
